# EUROPEAN PATENT SPECIFICATION

(11) **EP 3 429 602 B1**
(45) Date of publication and mention of the grant of the patent: **07.05.2025**
(21) Application number: 17767522.0
(22) Date of filing: 16.03.2017
(51) Int. Cl.: A61K 31/722, A61K 31/726, A61L 2/232, A61P 1/14

(54) **COMPOSITIONS COMPRISING A POLYGLUCOSAMINE-ARGININE AND THEIR USE TO TREAT DYSBIOSIS**
POLYGLUCOSAMINE-ARGININE ENTHALTENDE ZUSAMMENSETZUNGEN UND IHRE VERWENDUNG ZUR BEHANDLUNG VON DYSBIOSIS
COMPOSITIONS COMPRENANT UNE POLYGLUCOSAMINE-ARGININE ET LEUR UTILISATION POUR TRAITER LA DYSBIOSE

(30) Priority: 16.03.2016 US 201662309281 P; 17.03.2016 US 201662309734 P
(43) Date of publication of application: 23.01.2019
(73) Proprietor: Synedgen, Inc., Claremont, CA 91711 (US)
(72) Inventor: BAKER, Shenda, Upland, CA 91784 (US); WIESMANN, William, P., Chevy Chase, MD 20815 (US); TOWNSEND, Stacy, Marie, Rancho Cucamonga, CA 91730 (US)
(74) Representative: Marks & Clerk LLP
(86) International application number: PCT/US2017/022698
(87) International publication number: WO 2017/161113

(56) References cited:
- WO-A1-2011/028967
- WO-A1-2016/040899
- WO-A2-2007/142704
- WO-A2-2015/095241
- CN-A- 1 965 650
- US-A1- 2009 202 430
- US-A1- 2010 130 443
- US-A1- 2011 206 654
- US-A1- 2013 164 311
- US-A1- 2013 210 761
- US-A1- 2014 080 785
- US-B1- 6 323 189

## Description

### CLAIM OF PRIORITY

This application claims priority to U.S. Application No. 62/309,734, filed March 17, 2016, and U.S. Application No. 62/309,281, filed March 16, 2016.

### BACKGROUND

The mucosal surface of the intestinal tract is a complex ecosystem combining the gastrointestinal epithelium, immune cells and resident microbiota. The mucosa of the intestinal tract is exposed to various microbial pathogens. These potentially harmful enteric microorganisms can hijack the cellular molecules and signaling pathways of the host and become pathogenic. The host is protected from attack by potentially harmful enteric microorganisms by the physical and chemical barriers created by the intestinal epithelium. The GI epithelia also have a thick glycocalyx (Navabi 2013) and with mucins provide an innate barrier to pathogen invasion and provide a suitable environment for commensal bacteria to maintain healthy gut flora (Linden 2008).

Most healthy epithelial surfaces are associated with a large population of normally nonpathogenic bacteria, known as commensal bacteria or the microbiota, that compete with pathogenic microorganisms for nutrients and for attachment sites on epithelial cells. Commensals also help to strengthen the barrier functions of epithelia by stimulating the epithelial cells to produce antimicrobial peptides. Under some circumstances commensal microbes themselves can cause disease if their growth is not kept in check or if the immune system is compromised. The survival of commensal microorganisms on mucosal surfaces is regulated by a balance between bacterial growth and their elimination by the mechanisms of innate and adaptive immunity.

The composition of the microbiota is influenced by the stability of diversity of species over time and re-establishment of health (homeostasis) after disease or damage. The human microbiota has co-evolved with hosts and influenced by host factors, primarily associated with infection or inflammation. Human health is influenced by altering the mucosal surfaces including the epithelial cells, the glycocalyx barrier and the mucins and thus altering the microbiota by exposure to chemical, radiation, physical damage, antibiotic treatment, or genetic defects that alter host factors that shape the microbiota [van der Waaj, 1971; Mazmanian, 2008]. During many disease states, a loss in microbial diversity, dysbiosis, is observed. Dysbiosis has been associated with inflammatory bowel disease, specifically with a reduction in biodiversity, the decreased representation of different taxa in the Firmicutes phylum and an increase in Gammaproteobacteria [Martiny, 2015]. Redundant mechanisms of action exist within the host-microbiota ecosystem to support resilience and diversity of the microbiome maintenance of homeostasis to protect the host from dysbiosis.

Gastrointestinal infections, such as those from *C. difficile, Salmonella* or *Campylobacter,* take advantage of the hosts' natural defenses in order to overcome the innate immune response and establish a foothold in the gastrointestinal tract (GI tract) and displace commensal bacteria.

There have been several methods proposed for treating diseases of the GI tract and diseases associated with complications in the GI tract. For example, WO2011028967 describes methods of disrupting a biofilm in a subject by administering chitosan and WO2007142704 describes using chitosan to modulate microbial populations in animals. Meanwhile, WO2016040899 describes treating pulmonary diseases which can be associated with GI tract complications with polyglucosamine.

### SUMMARY OF THE INVENTION

The invention is described in the independent claim, with further features of the invention being recited in the dependent claims.

Any reference to methods of treatment throughout the description are to be interpreted as reference to the compound and compositions of the present invention for use in such methods.

Provided herein are methods of use of a broadly applicable, host-targeted, mucosal drugs to enhance and mimic the host response to deleterious mucosal stimulus or damage and to behave in a manner similar to the hosts own defenses, for example controlled by dose and timing of administration. For example, provided in the disclosure herein are methods of use of polycationic polysaccharides (e.g., polyglucosamines or derivatized polyglucosamines) as host targeted therapeutic agents that act at the mucosal surfaces in the gastrointestinal tract (GI tract), e.g., the oral cavity, throat, esophagus, stomach, upper and lower intestines, colon) to mimic and enhance the host ability to 1) prevent and treat infection from pathogenic bacteria (alone or with synergy; reduce antimicrobial resistance); 2) prevent and reduce inflammation from deleterious mucosal stimulus (pathogen, heat, damage, disease, radiation) and to restore homeostasis to bowel inflammation (alone or with anti-inflammatories); 3) prevent and treat obstructive GI syndromes; 4) to restore homeostasis to the GI tract of the microflora.

In one aspect, provided is a method of treating (e.g., inhibiting, modulating, reversing, or reducing the severity of at least one symptom of) a subject having dysbiosis, the method comprising administering an effective amount of a composition comprising polyglucosamine-arginine (PAAG) of the Formula (I): 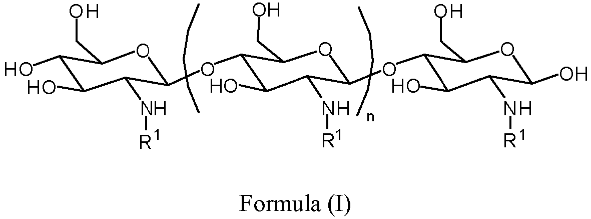 wherein: n is an integer between 20 and 6000 and each R¹ is independently selected for each occurrence from hydrogen, acetyl, 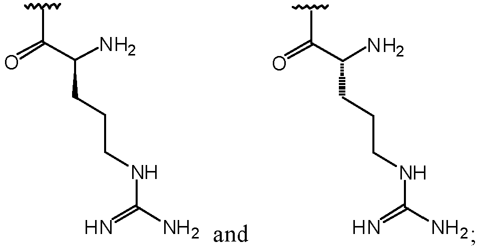 wherein at least 25% of R¹ substituents are H, and at least 2% of R¹ substituents are 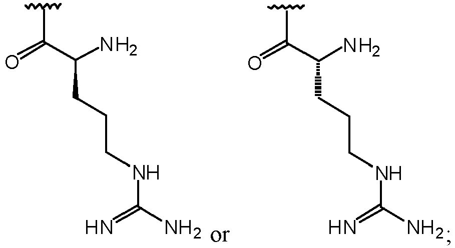 thereby treating the subject.

In some embodiments, at least 0.1% of R¹ substituents are acetyl. In some embodiments, at least 0.5%, at least 1%, at least 2%, at least 3%, at least 4%, or at least 5% of the R¹ substituents are acetyl. In some embodiments, at least 1% of R¹ substituents are acetyl.

In some embodiments, the method comprises administering to the subject a polyglucosamine-arginine (PAAG) of the Formula (I): 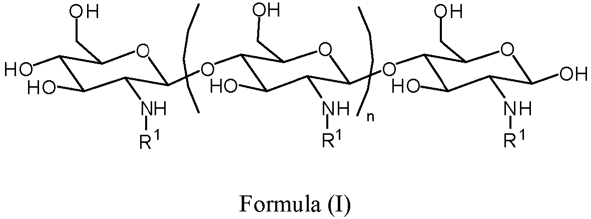 wherein: n is an integer between 20 and 6000; and each R¹ is independently selected for each occurrence from hydrogen, acetyl, 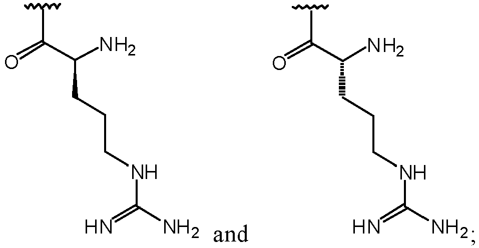 wherein at least 25% of R¹ substituents are H, at least 1% of R¹ substituents are acetyl, and at least 2% of R¹ substituents are 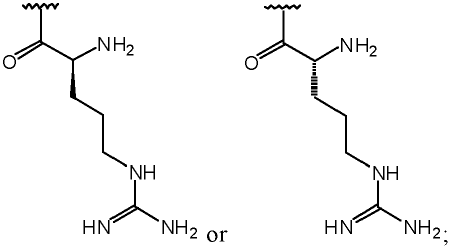 thereby treating the subject.

In some aspects, the method treats inflammation or infection (e.g., inflammation or infection resulting in dysbiosis).

In one aspect of the disclosure, provided is a method of treating a subject having disease or disorder of the gastrointestinal tract (e.g., gastroenteritis, DIOS), or a symptom or complication thereof, the method comprising administering to the subject a polyglucosamine-arginine (PAAG) of the Formula (I): 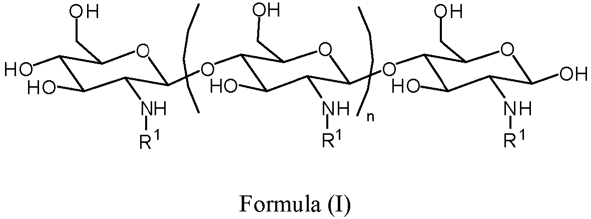 wherein: n is an integer between 20 and 6000; and each R¹ is independently selected for each occurrence from hydrogen, acetyl, 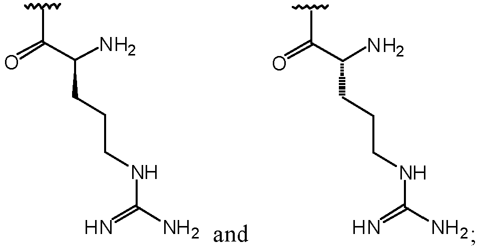 wherein at least 25% of R¹ substituents are H, and at least 2% of R¹ substituents are 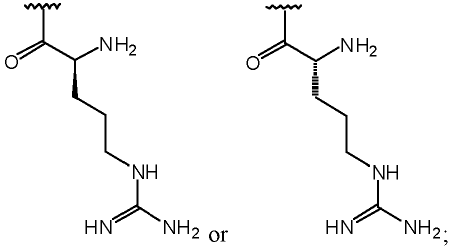 thereby treating the subject.

In some embodiments, at least 0.1% of R¹ substituents are acetyl. In some embodiments, at least 0.5%, at least 1%, at least 2%, at least 3%, at least 4%, or at least 5% of the R¹ substituents are acetyl). In some embodiments, at least 1% of R¹ substituents are acetyl.

In some embodiments, the method comprises administering to the subject a polyglucosamine-arginine (PAAG) of the Formula (I): 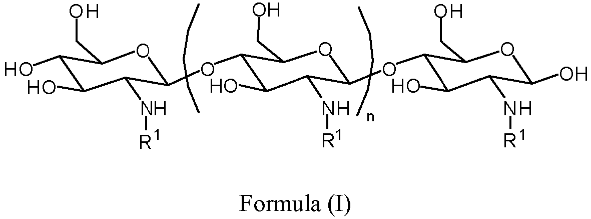 wherein: n is an integer between 20 and 6000; and each R¹ is independently selected for each occurrence from hydrogen, acetyl, 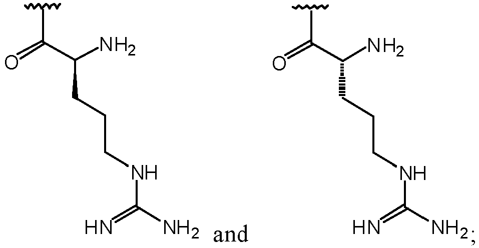 wherein at least 25% of R¹ substituents are H, at least 1% of R¹ substituents are acetyl, and at least 2% of R¹ substituents are 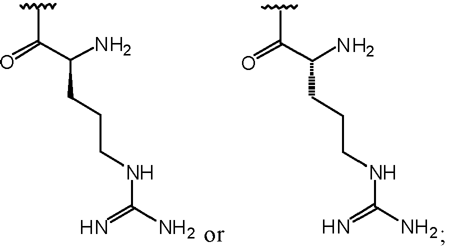 thereby treating the subject.

In some aspects of the disclosure, the methods described herein act at the mucosal surfaces in the GI tract (oral, throat, esophagus, stomach, upper and lower intestines, colon) to mimic and enhance the host ability to 1) prevent and treat infection from pathogenic bacteria (alone or with synergy; reduce antimicrobial resistance); 2) prevent and reduce inflammation from deleterious mucosal stimulus (pathogen, heat, damage, disease, radiation) and to restore homeostasis to bowel inflammation (alone or with anti-inflammatories); 3) prevent and treat obstructive GI syndromes; or 4) to restore homeostasis to the GI tract of the microflora.

### BRIEF DESCRIPTION OF THE DRAWINGS

**FIG. 1** depicts a reduction in the mean endoscopy proctitis scores on Day 7 on the radiation proctitis model following twice daily rectal treatment with PAAG.
**FIGS. 2A-2B** depict a reduction in local and systemic biomarkers for inflammation in the radiation proctitis model following twice daily rectal treatment with PAAG.
**FIGS. 3A-3B** depict a reduction in local and systemic biomarkers for inflammation in the radiation proctitis model following daily oral treatment with PAAG.
**FIG. 4** depicts a reduction in necrotic enteritis and mortality by treatment with PAAG compared to vehicle treated controls in a coccidia and *Clostridium perfringens* infection model.
**FIG. 5** depicts mouse ileal tissue differential gene expression changes (red up regulated; green down regulated) in TB irradiated mice 4 days following treatment with 50 mg/kg PAAG daily relative to vehicle control.

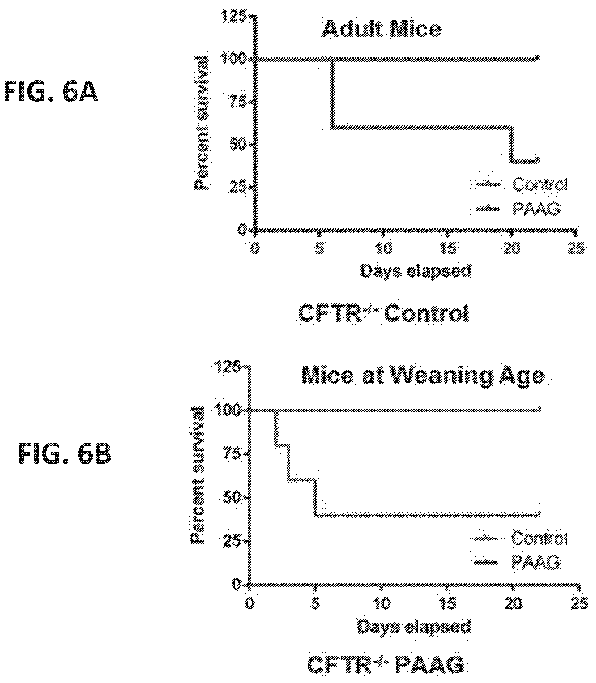

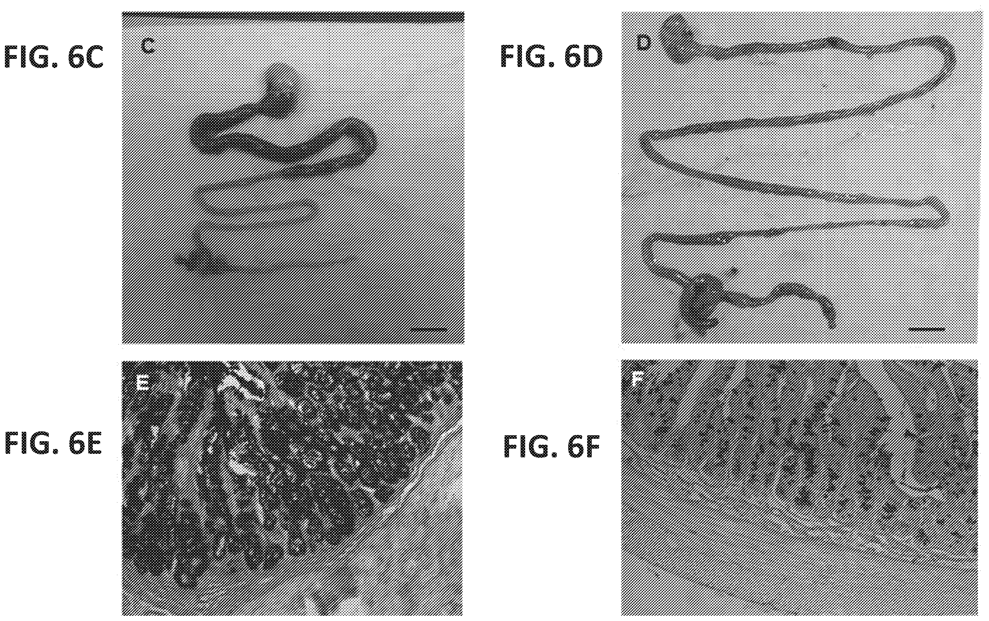

**FIG. 6A-6F** depict GI obstruction prevented by PAAG therapy in mice. **FIG. 6A****:** Adult mice give PAAG (40 mg/kg/d) by oral gavage once daily for 21 days immediately after transition to a regular diet; **FIG. 6B****:** Mice at weaning age were given PAAG (40 mg/kg/d) by oral gavage divided three times daily for 21 days while initiated on a regular diet; **FIGS. 6C-6D****:** Representative images of gross intestine of control (**FIG. 6C**) and PAAG treated (**FIG. 6D**) mice; **FIGS. 6E-6F****:** PAS staining of control (**FIG. 6E**) and PAAG treated (**FIG. 6F**) intestine showing significant improvement of mucus impaction with PAAG treatment.
**FIGS. 7A-7B** depict the fecal calprotectin and serum procalcitonin levels for PAAG treated mice after acute radiation on day 0 inducing proctitis.
**FIG. 8** depicts the mean percent weight change over 5 days.
**FIG. 9** depicts mean colitis scores from animals day 5.
**FIGS. 10A-10D** depict representative endoscopy images from animals with treatment with PAAG.
**FIG. 11** depicts mean inflammation scores from colon tissue from animals sacrificed day 5.
**FIG. 12** depicts mean edema scores from colon tissue from animals sacrificed day 5.
**FIG. 13** depicts mean sum pathology scores from colon tissue from animals sacrificed day 5.
**FIGS. 14A-14D** depicts representative pathology images from colon tissue from animals sacrificed day 5.

### DETAILED DESCRIPTION OF CERTAIN EMBODIMENTS OF THE INVENTION

In one aspect, provided is a method of treating (e.g., inhibiting, modulating, reversing, or reducing the severity of at least one symptom of) a dysbiosis in a subject, the method comprising administering an effective amount of a composition comprising polyglucosamine-arginine (PAAG) of the Formula (I): 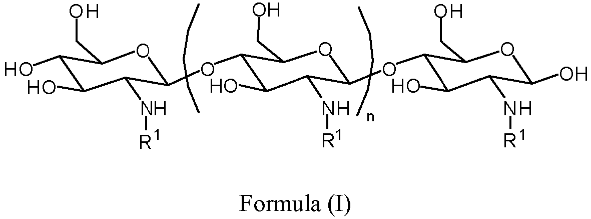 wherein: n is an integer between 20 and 6000; and each R¹ is independently selected for each occurrence from hydrogen, acetyl, 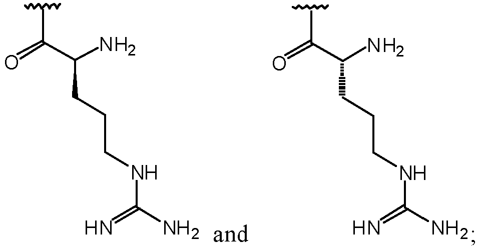 wherein at least 25% of R¹ substituents are H, and at least 2% of R¹ substituents are 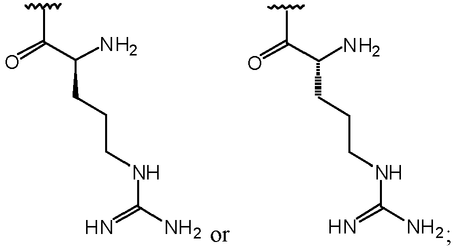 thereby treating the subject.

In some embodiments, at least 0.1% of R¹ substituents are acetyl. In some embodiments, at least 0.5%, at least 1%, at least 2%, at least 3%, at least 4%, or at least 5% of the R¹ substituents are acetyl. In some embodiments, at least 1% of R¹ substituents are acetyl.

In some embodiments, the method comprises administering an effective amount of a composition comprising polyglucosamine-arginine (PAAG) of the Formula (I) 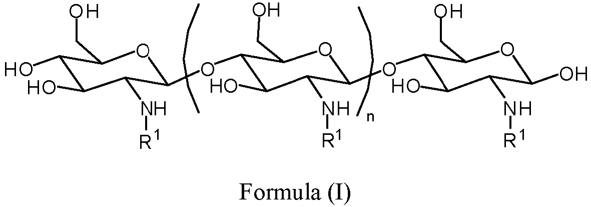 wherein: n is an integer between 20 and 6000; and each R¹ is independently selected for each occurrence from hydrogen, acetyl, 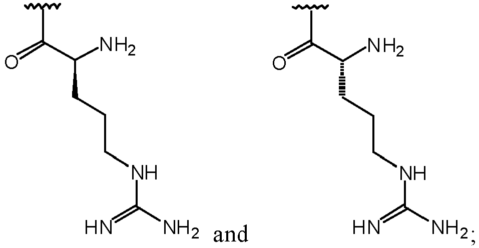 wherein at least 25% of R¹ substituents are H, at least 1% of R¹ substituents are acetyl, and at least 2% of R¹ substituents are 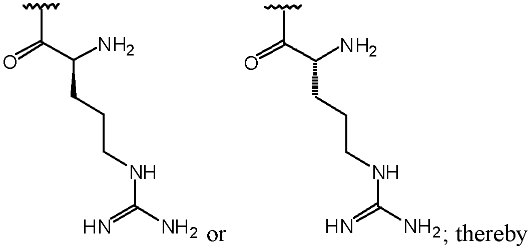 treating the subject. In some embodiments, the method results in reduction or elimination of at least one pathogen or pathobiont present in the GI tract of the subject. In some embodiments, the method results in augmentation or growth of at least one type of bacteria (e.g., at least one type of bacteria no detectably present in the composition or in the GI tract prior to administration). In some embodiments, the method provides microbiome homeostasis. In some embodiments, the method modulates the microbiota diversity present in the GI tract. In some embodiments, the method maintains or balances the diversity of the commensal microflora. In some embodiments, the amount of bacteria present in the GI tract (e.g., commensal populations) is not reduced (e.g., by 5, 10, 15, 20%) relative to a subject not administered the compound or composition described herein. In some examples, the population of *Enterococci* or *E. coli* does not increase (e.g., relative to a subject not treated with the compound). In some examples, the population of *Enterococci* or *E. coli* decreases (e.g., relative to a subject not treated with the compound). In some embodiments, the method reduces dissemination of bacteria (reduces by 20% inflammation scores from histology) to distal organs (e.g., liver, spleen, lymph nodes). In some embodiments, the method provides therapeutic effect within 7, 14, 21, 28 days.

In some embodiments, the dysbiosis is a result of an allergic effect. In some embodiments, the dysbiosis is a result of an autoimmune and inflammatory disorder. In some embodiments, the dysbiosis is a result of celiac disease.

In some examples, the symptom is diarrhea, vomiting, fever, or abdominal cramps.

In some examples, the complication is colitis, sepsis, meningitis, or kidney failure.

In one aspect of the disclosure, provided is a method of treating a subject having disease or disorder of the gastrointestinal tract (e.g., gastroenteritis, DIOS), or a symptom or complication thereof, the method comprising administering to the subject a polyglucosamine-arginine (PAAG) of the Formula (I): 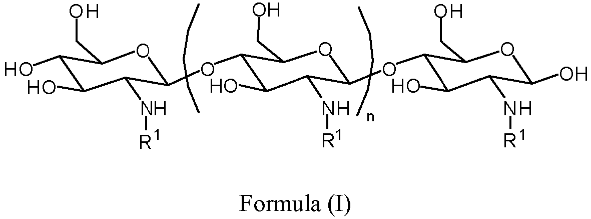 wherein: n is an integer between 20 and 6000; and each R¹ is independently selected for each occurrence from hydrogen, acetyl, 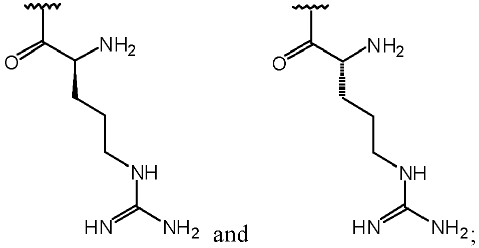 wherein at least 25% of R¹ substituents are H, and at least 2% of R¹ substituents are 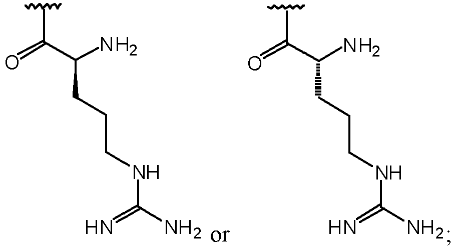 thereby treating the subject.

In some examples, at least 0.1% of R¹ substituents are acetyl. In some embodiments, at least 0.5%, at least 1%, at least 2%, at least 3%, at least 4%, or at least 5% of the R¹ substituents are acetyl. In some embodiments, at least 1% of R¹ substituents are acetyl.

In some examples, the method comprises administering to the subject a polyglucosamine-arginine (PAAG) of the Formula (I): 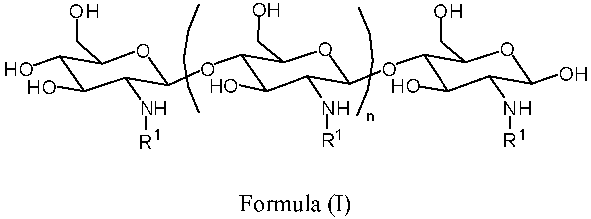 wherein: n is an integer between 20 and 6000; and each R¹ is independently selected for each occurrence from hydrogen, acetyl, 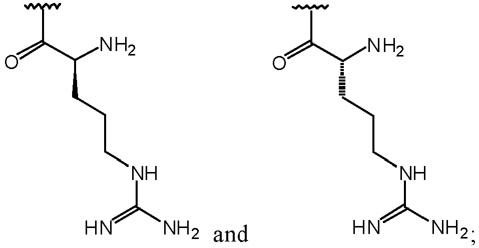 wherein at least 25% of R¹ substituents are H, at least 1% of R¹ substituents are acetyl, and at least 2% of R¹ substituents are 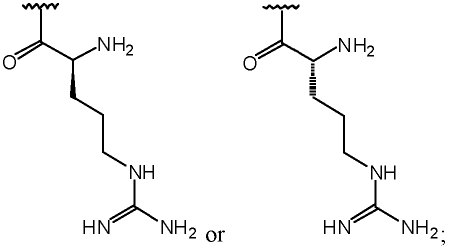 thereby treating the subject.

In some examples, the method decreases mortality by 5%, 10%, 15%, 20%, or 25%, relative to a subject not administered with the compound. In some examples , the method improves the GI transit time (e.g., relative to a subject not treated with the compound). In some examples , the method reduces mucus (e.g., the thick adherent mucus) in the subject (e.g., relative to a subject not treated with the compound). In some examples, the method reduces proinflammatory cytokine production (e.g., relative to a subject not treated with the compound). In some examples, the method reduces colon length (e.g., relative to a subject not treated with the compound). In some embodiments, the method reduces bacterial dissemination to distal tissues (e.g., liver, spleen, mesenteric lymph nodes) (e.g., relative to a subject not treated with the compound). In some examples, the method reduces inflammatory response (e.g., relative to a subject not treated with the compound). In some examples, the method reduces attachment, adhesion, invasion, or survival of a pathogen as described herein by at least 10, 20, 30%, 40%, 50%, 60%, 70%, 80%, 90%, 95%, or 99%, e.g., after 6, 8, 12, 16, 24 hour treatment, e.g., relative to a subject not treated with the compound. In some embodiments, the method reduces inflammation (e.g., as indicated by scores from histology (e.g., by at least 10, 20%, 30%, 40%, 50%, 60%, 70%, 80%, 90%, 95%, or 99%, e.g., relative to a subject not treated with the compound). In some examples , the method stimulates defensin production (e.g., by reducing cell death) (e.g., relative to a subject not treated with the compound). In some embodiments, the method reduces bacterial invasion into the GI mucosa (e.g., by stopping disruption of tight junctions). In some examples , the method augments barrier function (e.g., reduces mucosal barrier damage and GI dysbiosis). In some examples , the method mediates immunomodulatory host capabilities.

In some embodiments, the composition is administered concomitant to consumption of a food or beverage product. In some embodiments, the composition is administered prior to consumption of a food or beverage product. In some embodiments, the composition is administered following consumption of a food or beverage product.

In some embodiments, the method controls growth or colonization of the GI by one or more pathogenic bacterium.

In some embodiments, the method further comprises administration of an additional agent. In some embodiments, the agent is synergistic with the compound.

In some embodiments, the composition (e.g., composition as described herein, e.g., composition comprising a compound described herein) is delivered orally (e.g., as a dry product (e.g., capsule, tablet) or aqueous solution). In some embodiments, the composition (e.g., composition as described herein, e.g., composition comprising a compound described herein) is delivered rectally (e.g., as an enema, suppository, via catheter, or via syringe).

In some embodiments, 10 to 5000 mg (e.g., 50 to 250 mg, 250 to 1000 mg) of the composition (e.g., the composition comprising a compound described herein) is administered (e.g., daily). In some embodiments, 0.1 µg to 1 mg (e.g., 1 µg to 1 mg, 0.1 µg to 500 µg, 1 µg to 500 µg) of the composition (e.g., the composition comprising a compound described herein) is administered (e.g., daily). In some embodiments, the composition is administered once daily (e.g., for 1, 2, 3, 4 weeks). In some embodiments, the composition (e.g., composition as described herein, e.g., composition comprising a compound described herein) is administered 1, 2, 3, or 4 times at 4 to 50 mg/kg/dose/day.

In some embodiments, the composition further comprises a carrier. In some embodiments, the composition additionally comprises 1, 2, or 3% w/w or w/v carrier (e.g., glycerol). In some embodiments, the composition additionally comprises from 1 to 2% (e.g., 1.38%) w/w or w/v glycerol. In some embodiments, the carrier is glycerol.

In some embodiments, the composition does not comprise a carrier (e.g., a non-fermentable sugar, e.g., glycerol or a non-fermentable sugar, e.g., trehalose or lactose).

In some embodiments, the polydispersity index of the composition (e.g., composition comprising PAAG) is from 1.0 to 2.5. In some embodiments, the pH of the composition is about 2 to about 10 (e.g., about 3 to about 9, about 2 to about 6, about 6 to about 9, or about 4 to about 8). In some embodiments, the pH of the composition is about 7 to about 8.

### Gastrointestinal dysbiosis

Provided herein are methods that modulate or perturb the microbiome environment, for example the methods described herein can restore normal diversity, homeostasis or function of the gut microbiome. In some embodiments, the methods described herein treat dysbiosis (e.g., GI dysbiosis). In some aspects of the disclosure that are not covered by the claimed invention, the methods described herein treat diseases or disorders in which the gut microbiome play an important role (e.g., obesity, type 2 diabetes, insulin resistance). In some examples, the methods described herein treat acute diseases or disorders as described herein. In someexamples, the methods described herein treat chronic diseases or disorders as described herein.

As used herein, "microbiome" refers to the genetic content of the communities of microbes that live in and on the human body, both sustainably and transiently, including eukaryotes, archaea, bacteria, and viruses (including bacterial viruses (i.e., phage)), wherein "genetic content" includes genomic DNA, RNA such as ribosomal RNA, the epigenome, plasmids, and all other types of genetic information. The microbiome is involved in the regulation of metabolic processes, including digestion, absorption, and conversion of indigestible foods or partially digested food ingredients to molecules that may signal physiological host mechanisms. A healthy microbiome provides the host with multiple benefits, including colonization resistance to a broad spectrum of pathogens, essential nutrient biosynthesis and absorption, and immune stimulation that maintains a healthy gut epithelium and an appropriately controlled systemic immunity. A change in the gut microbiome habitat may result in microbiota community shifts and consequential changes in glucose regulation (Nature 2012, v. 490, pg. 55-60). For example, an analysis of fecal microbiota showed that subjects with type 2 diabetes have a moderate degree of gut microbial dysbiosis, a decrease in the abundance of some universal butyrate producing bacteria, and an increase in various opportunistic pathogens, as well as an enrichment of other microbial functions conferring sulphate reduction and reduction of oxidative stress resistance. The microbiome may be characterized in healthy individuals and those inflicted with disease. In healthy individuals, the gut microbiome is defined as normal.

"Dysbiosis" refers to a state of the microbiome of the gut or other body area, including mucosal or skin surfaces in which the normal diversity or function of the ecological network is disrupted. Any disruption from the preferred (e.g., ideal) state of the microbiota can be considered a dysbiosis, even if such dysbiosis does not result in a detectable decrease in health. This state of dysbiosis may be unhealthy, it may be unhealthy under only certain conditions, or it may prevent a subject from becoming healthier. Dysbiosis may be due to a decrease in diversity, the overgrowth of one or more pathogens or pathobionts, symbiotic organisms able to cause disease only when certain genetic or environmental conditions are present in a patient, or the shift to an ecological network that no longer provides a beneficial function to the host and therefore no longer promotes health. Dysbiosis may be induced by illness, treatment (e.g., overuse) with an agent (e.g., antibiotic, e.g., antibiotic reducing commensal flora), or other environmental factors. In settings of 'dysbiosis' or disrupted symbiosis, microbiome functions can be lost or deranged, resulting in increased susceptibility to pathogens, altered metabolic profiles, or induction of proinflammatory signals that can result in local or systemic inflammation or autoimmunity. Thus, the intestinal microbiome plays a significant role in the pathogenesis of many diseases and disorders, including a variety of pathogenic infections of the gut. For instance, subjects become more susceptible to pathogenic infections when the normal intestinal microbiota has been disturbed due to use of broad-spectrum antibiotics. Many of these diseases and disorders are chronic conditions that significantly decrease a subject's quality of life and can be ultimately fatal.

In some cases, the dysbiosis results in increased fecal pH, increased production of hydrogen sulfide and methane gases, reduced antioxidant capacity, presence of opportunistic microbiota, presence of pathogenic fungi and yeast, increased intestinal inflammation, decreased intestinal mucosal thickness, colon ulcers and leaky gut. Improvements may be observed from decreased fecal pH, decreased production of hydrogen sulfide and methane gases, increased antioxidant capacity, absence of opportunistic microbiota, absence of pathogenic fungi and yeast, decreased intestinal inflammation, normal intestinal mucosal thickness, healthy colon anatomy and less circulating immunoglobulin A antibodies. Dysbiosis in subjects may be identified by profiling (e.g., sequencing to identify the bacteria present in the subject's microbiota). In some embodiments, subjects are identified with protein and gene markers of inflammation (e.g., fecal calprotectin, plasma procalcitonin).

The intestinal gut microbiota provides many crucial functions to its host, including contribution to digestion, the development of the immune system, and resistance to pathogenic colonization. Even a slight fluctuation in the symbiotic balance may be deleterious to the host, leading to pathological conditions such as, e.g., *Clostridium difficile* infection of IBD. Exemplary conditions relating to dysbiosis include a condition of the gut, inflammatory bowel diseases (IBD), Crohn's Disease, IBS, stomach ulcers, colitis, neonatal necrotizing enterocolitis, gastroesophageal reflux disease (GERD), gastroparesis, CF, COPD, rhinitis, atopty, asthma, acne, allergies (e.g., food allergy), obesity, periodontal disease, diarrhea, constipation, functional bloating, gastritis, lactose intolerance, visceral hyperalgesia, colic, pouchitis, diverticulitis, sinusitis, COPD, depression, ADHD, autism, Alzheimers, migranes, MS, Lupus, arthritis, Type 2 diabetes, obesity, non alcoholic steato hepatitis (NASH), non alcoholic fatty liver disease (NAFLD), risk of infarction/cardiovascular risk, heart failure, cancer, dental caries, gingivitis, oral cancer, oral mucosistis, bacterial vaginosis, fertility, lung cancer, psoriasis, atopic dermatitis, MRSA, combinations thereof. The microbiome also play a role in metabolic disease, disturbances in the probiotic activity, protection against cell injury, regulation of host fat storage, stimulation of intestinal angiogenesis and the like.

The methods described in the present disclosure may protect or otherwise provide therapeutic effect against infection by one or more GI pathogens, and therefore be administered after an acute case of infection has been resolved (e.g., in order to prevent relapse, during an acute cases of infection as a complement to antibiotic therapy if the composition is not sensitive to the same antibiotics as the GI pathogen, or to prevent infection or reduce transmission from disease carriers). Exemplary pathogens include, but are not limited to *Aeromonas hydrophila, Campylobacter fetus, Plesiomonas shigelloides, Bacillus cereus, Campylobacter jejuni, Clostridium botulinum, Clostridium difficile, Clostridium perfringens,* enteroaggregative *Escherichia coli,* entero hemorrhagic *Escherichia coli,* enteroinvasive *Escherichia coli,* enterotoxigenic *Escherichia coli* (LT and/or ST), *Escherichia coli* 0157:H7, *Helicobacter pylori, Klebsiella pneumonia, Lysteria monocytogenes, Plesiomonas shigelloides, Salmonella spp., Salmonella typhi, Shigella spp., Staphylococcus, Staphylococcus aureus,* vancomycin-resistant *Enterococcus spp., Vibrio spp., Vibrio cholera, Vibrio parahaemolyticus, Vibrio vulnificus,* and *Yersinia enterocolitica.*

In some examples , the subjects treated with the methods described herein have less severe diarrhea, less abdominal pain, less bloating, less loose stool, less nausea, less heartburn, less stomach cramps, and decreased fecal pH, as compared to subjects not treated with the methods described herein. In some examples, the subjects treated with the methods described herein have partial or complete alleviation, amelioration, relief, inhibition, delaying onset, reducing severity or incidence of symptoms (e.g., symptoms of a disease or disorder described herein, for example a gastrointestinal tract infection, e.g., IBD). In some examples, IBD is ulcerative colitis (UC) and Crohn's disease (CD).

In some aspects of the disclosure, the methods described herein treat pathogenic overgrowth. In some embodiments, the methods described herein treat systemic inflammation or bacterial translocation.

In some aspects of the disclosure not covered by the claimed invention, the methods described herein treat stress. In some aspects , the methods described herein treat infection, inflammation, or obstruction resulting from oral drug or alcohol use. In some aspects of the disclosure not covered by the claimed invention, the methods described herein treat chronic fatigue syndrome, or obesity (e.g., due to infection, inflammation, or obstruction).

In some embodiments, the methods described herein treat dysbiosis caused by or associated with overuse of antibiotics (reducing commensal flora), inflammation (e.g., IBD, colitis), other diseases (e.g., chronic fatigue syndrome, obesity), pathogenic overgrowth, systemic inflammation and bacterial translocation, stress, or oral drugs or alcohol.

In some aspects of the disclosure not covered by the claimed invention, the methods described herein improves weight gain in a subject (e.g., a human subject).

### Gastrointestinal tract infections

In some aspects of the disclosure the methods described herein can be used to treat or prevent gastrointestinal tract infections in a subject. For example, liquid or solid particulate compositions comprising soluble polyglucosamines or derivatized polyglucosamines described herein can be used to treat or prevent gastrointestinal tract infections, e.g., gastrointestinal tract bacterial infections, in a subject. Treatment or prevention includes administration of soluble polyglucosamines or derivatized polyglucosamines alone or in combination with drugs or treatments described below.

Gastrointestinal tract infections can be caused by e.g., bacteria (e.g., enteric bacteria), viruses, parasites or fungi. Exemplary gastrointestinal tract bacterial infections include noninflammatory gastroenteritis caused by e.g., *Staphylococcus aureus, Bacillus cereus, Clostridium perfringens, Clostridium difficile* or *Clostridium botulinum;* inflammatory gastroenteritis caused by e.g., *Vibrio cholerae,* Enterotoxigenic (ETEC) *Escherichia coli,* Enteropathogenic (EPEC) *Escherichia coli,* Enteroaggregative (EAggEC) *Escherichia coli, Clostridium dificile, Vibrio parahemolyticus,* or *Bacillus anthracis;* or invasive gastroenteritis caused by e.g., *Shigella sp., Salmonella sp., Campylobacter jejuni,* Enteroinvasive (EIEC) *Escherichia coli,* Enterohemorrhagic (EHEC) *Escherichia coli, Vibrion vulnificus, Yersinia sp., Francisella tularensis,* or *Helicobacter pylori.*

Symptoms of gastrointestinal tract infections include, e.g., diarrhea, vomiting, abdominal pain, cramps, fecal leukocytes, fever, dysentery, and/or blood in stool.

Gastrointestinal tract infections can be treated or prevented using soluble polyglucosamines or derivatized polyglucosamines described herein, in combination with one or more of agents or therapeutics. Exemplary agents and therapeutics to treat gastrointestinal tract infections includes rehydration, dietary therapy, probiotics, zinc, pharmacologic therapy (e.g., antibiotics (e.g., fluoroquinolone, metronidazole or vancomycin), antidiarrheal agents (e.g., loperamide or bismuth subsalicylate (BSS)), or antiemetic drugs (e.g., ondansetron or metoclopramide)). In some embodiments, the administrations of a combination of agents and therapeutics are spaced sufficiently close together such that a synergistic effect is achieved.

In some aspects of the disclosure, the methods described herein have partial or complete alleviation, amelioration, relief, inhibition, delaying onset, reducing severity or incidence of symptoms (e.g., symptoms of a disease or disorder described herein, for example a total body ionizing radiation or acute radiation syndrome (e.g., radiation or chemotherapy induced inflammatory disease (e.g., radiation proctitis, GI mucositis)).

### Diverticulitis

In some aspects of the disclosure , the methods described herein have partial or complete alleviation, amelioration, relief, inhibition, delaying onset, reducing severity or incidence of symptoms of diverticulitis. Diverticulitis occurs when pouches (diverticula) form in the wall of the colon and become inflamed or infected (e.g., from bacterial growth in the diverticula).

### Pouchitis

In some aspects of the disclosure, the methods described herein have partial or complete alleviation, amelioration, relief, inhibition, delaying onset, reducing severity or incidence of symptoms of pouchitis. Pouchitis refers to inflammation of the ileal pouch (an artificial rectum surgically created out of ileal gut tissue in subjects who have undergone a colectomy), which is created in the management of subjects with ulcerative colitis, indeterminate colitis, FAP, or colitides.

### Gastritis

In some aspects of the disclosure, the methods described herein have partial or complete alleviation, amelioration, relief, inhibition, delaying onset, reducing severity or incidence of symptoms (e.g., symptoms of a disease or disorder described herein, for example gastritis. Gastritis refers to irritation from excessive alcohol use, chronic vomiting, stress, or use of certain medications (e.g., aspirin or NSAIDs).

### Distal Intestinal Obstructive Syndrome (DIOS)

Distal intestinal obstruction syndrome (DIOS) often occurs in individuals with cystic fibrosis and involves the blockage of intestines by thickened stool. In individuals with cystic fibrosis, mucus builds up along the intestinal tract and slows the emptying of food. The resultant build-up of stool behind the mucus-filled area causes blockage (e.g., obstructed with mucofaeculent material in the distal ileum and right colon). DIOS is similar to constipation (e.g., there is a back-up of stool in the digestive tract), but the back-up of stool is higher up in the intestines. DIOS in newborn infants is also referred to as meconium ileus equilavent. Symptoms of DIOS include inspissated intestinal secretions, pancreatic insufficiency, undigested food residue, disordered intestinal motility, faecal stasis, dehydration, abdominal pain, vomiting, and palpable mass in the abdomen. DIOS treatment typically requires surgery to relieve the obstruction, especially when there is sign of bowel rupture. More conservative approaches may be attempted, including restricting oral intake, placement of a nasogastric tube for decompression of the stomach and proximal intestines, and laxative and enema administration. Individuals suffering from DIOS tend to have repeat episodes, often requiring maintenance therapy with pancreatic enzyme replacement and stool softeners. In some aspects of the disclosure, the methods described herein have partial or complete alleviation, amelioration, relief, inhibition, delaying onset, reducing severity or incidence of symptoms of DIOS.

### Meconium Ileus

Meconium ileus is a condition where a baby's first stool (i.e., meconium) is blocking the last part of the small intestine. Meconium ileus can happen when the meconium is thicker and more sticky than normal. The small intestine can become enlarged, loops of small intestine may distend, or push out, the abdomen. Below the blackage, the large intestine is narrow. It may be empty, or may hold small pellets of dried meconium or plugs of mucus from the lining of the intestine. Almost all babies with meconium ileus have cystic fibrosis (CF). CF makes certain fluids and mucus in the body thicker than normal.

In some aspects of the disclosure that are not encompassed by the claims, the methods described herein may be used to treat a subject suffering from meconium ileus.

### Paralytic ileus

Paralytic ileus refers to obstruction of the intestine due to paralysis of the intestinal muscles. For example, the intestinal muscles can become so inactive that it prevents the passage of food, leading to functional blockage of the intestine. Ileus may follow some types of surgery (e.g., abdominal surgery). Paralytic ileus can also result from drugs, injury, or inflammation within the abdomen that touches the intestines; or diseases of the intestinal muscles themselves.

### Necrotizing Entercolitis (NEC)

Necrotizing Entercolitis (NEC) is inflammation and death of intestinal tissue typically involving the lining of the intestine or the entire thickness of the intestine. In severe cases, the intestine may perforate and a hole develops in the intestinal wall. In cases when a hole develops in the intestinal wall, bacteria found in the intestine can leak into the abdomen and cause widespread infection (e.g., bacteria and other waste products can pass through the intestine and enter the baby's bloodstream or abdominal cavity). NEC is most common in premature infants, typically developing within two weeks of birth. However, NEC may occur up to three months after birth. Symptoms of NEC includes bloody stool, diarrhea, constipation, chills or fever, poor feeding, and vomiting. Current treatment options include intravenous feeding, antibiotics, and a tube that goes in the nose to the stomach to remove extra fluids and gas from the intestine. In some aspects of the disclosure that are not encompassed by the claims, the methods described herein have partial or complete alleviation, amelioration, relief, inhibition, delaying onset, reducing severity or incidence of symptoms of NEC.

### Short Bowel Syndrome (SBS)

Short Bowel Syndrome (SBS) is a malabsorption disorder caused by the surgical removal of the small intestine or due in rare cases to complete dysfunction of a large segment of the bowel. SBS is typically acquired, but some children are born with a congenital short bowel. SBS generally does not develop unless more than two thirds of the small intestine has been removed. SBS is usually caused by surgery for Crohn's disease, volvulus, tumors of the small intestine, injury or trauma to the small intestine, necrotizing enterocolitis, bypass surgery to treat obesity, or other surgeries to remove diseases or damaged portions of the small intestine.

### Compounds

### Soluble polyglucosamines and polyglucosamines derivatives

Compounds and compositions (*e.g.*, vacuum-dried, lyophilized, spray-dried, reconstituted) containing a soluble polyglucosamine or a derivatized polyglucosamine such as PAAG for treating (e.g., inhibiting, modulating, reversing, or reducing the severity of at least one symptom of) a dysbiosis in a subject (*e.g.*, a subject as described herein) are described herein.

Polyglucosamines can be derived from chitin or chitosan. Chitosan is an insoluble polymer derived from the deacetylation of chitin, which is a polymer of N-acetylglucosamine, that is the main component of the exoskeletons of crustaceans (*e.g.*, shrimp, crab, lobster). Chitosan is generally a β 1->4) polyglucosamine that is less than 50% acetylated while chitin is generally considered to be more than 50% acetylated. Polyglucosamines are also found in various fungi and arthropods. Synthetic sources and alternate sources of β 1->4) polyglucosamines may serve as the starting material for polyglucosamine derivatives. Polyglucosamines, as opposed to polyacetylglucosamines, are defined herein to be less than 50% acetylated. If greater than 50% of the amino groups are acetylated, the polymer is considered a polyacetylglucosamine.

A soluble polyglucosamine described herein refers to a neutral pH, water soluble polyglucosamine or polyglucosamine that is not derivatized on the hydroxyl or amine moieties other than with acetyl groups. A soluble polyglucosamine is comprised of glucosamine and acetylglucosamine monomers. Generally, a water soluble polyglucosamine (at neutral pH) has a molecular weight of less than or equal to about 5,000 kDa and a degree of deacetylation equal to or greater than 80%.

A polyglucosamine derivative described herein is generated by functionalizing the free hydroxyl or amine groups with positively charged or neutral moieties. The percent of functionalization is defined as the total percent of monomers on the polyglucosamine backbone that have been functionalized with a positively charged or neutral moiety. The degrees of deacetylation and functionalization impart a specific charge density to the functionalized polyglucosamine derivative. The resulting charge density affects solubility and effectiveness of treatment. Thus, in accordance with the present invention, the degree of deacetylation, the functionalization and the molecular weight must be optimized for optimal efficacy. The polyglucosamine derivatives described herein have a number of properties which are advantageous, including solubility at physiologic (neutral) pH. In some embodiments, the polyglucosamine derivative is soluble up to a pH of 10. In some embodiments, the molecular weight (e.g., i.e., weight average molecular weight) of the polyglucosamine derivative is between 5 and 1,000 kDa. In some embodiments, the molecular weight (i.e., weight average molecular weight) of the polyglucosamine derivative is between 15 and 1,000 kDa. In some embodiments, the molecular weight of the polyglucosamine derivative is between 20 and 450 kDa. In some embodiments, the molecular weight (i.e., weight average molecular weight) of the polyglucosamine derivative is between 20 and 350 kDa. In some embodiments, the molecular weight (i.e., weight average molecular weight) of the polyglucosamine derivative is between 25 and 200 kDa. The polyglucosamine derivative described herein is soluble at pH 2 to pH 11.

Polyglucosamines with any degree of deacetylation (DDA) greater than 50% are used in the present invention, with functionalization between 2% and 50% of the total monomers on the polyglucosamine backbone. The degree of deacetylation determines the relative content of free amino groups to total monomers in the polyglucosamine polymer. Methods that can be used for determination of the degree of deacetylation of polyglucosamine include, *e.g.,* ninhydrin test, linear potentiometric titration, near-infrared spectroscopy, nuclear magnetic resonance spectroscopy, hydrogen bromide titrimetry, infrared spectroscopy, and first derivative UV-spectrophotometry. Preferably, the degree of deacetylation of a soluble polyglucosamine or a derivatized polyglucosamine described herein is determined by quantitative infrared spectroscopy.

Percent functionalization by active derivitization of the amines is determined relative to the total number of monomers on the polyglucosamine polymer. Preferably, the percent functionalization of a derivatized polyglucosamine described herein is determined by H-NMR or quantitative elemental analysis. The degrees of deacetylation and functionalization impart a specific charge density to the functionalized polyglucosamine derivative. The resulting charge density affects solubility, and strength of interaction with tissue, biofilm components and bacterial membranes. The molecular weight is also an important factor in a derivatized polyglucosamine's mucoadhesivity and biofilm disrupting capability. Thus, in accordance with the present invention, these properties must be optimized for optimal efficacy. Exemplary polyglucosamine derivatives are described in U.S.P.N. 8,119,780.

The polyglucosamine derivatives described herein have a range of polydispersity index (PDI) between about 1.0 to about 2.5. As used herein, the polydispersity index (PDI), is a measure of the distribution of molecular weights in a given polymer sample. The PDI calculated is the weight averaged molecular weight divided by the number averaged molecular weight. This calculation indicates the distribution of individual molecular weights in a batch of polymers. The PDI has a value always greater than 1, but as the polymer chains approach uniform chain length, the PDI approaches unity (1). The PDI of a polymer derived from a natural source depends on the natural source (e.g. chitin or chitosan from crab vs. shrimp vs. fungi) and can be affected by a variety of reaction, production, processing, handling, storage and purifying conditions. Methods to determine the polydispersity include, *e.g.,* gel permeation chromatography (also known as size exclusion chromatography); light scattering measurements; and direct calculation from MALDI or from electrospray mass spectrometry. Preferably, the PDI of a soluble polyglucosamine or a derivatized polyglucosamine described herein is determined by HPLC and multi angle light scattering methods.

The polyglucosamine derivatives (*i.e.,* derivatized polyglucosamines) described herein have a variety of selected molecular weights that are soluble at neutral and physiological pH, and include for the purposes of this invention molecular weights ranging from 5 - 1,000 kDa. Derivatized polyglucosamines are soluble at pH up to about 10. Embodiments described herein are feature medium range molecular weight of derivatized polyglucosamines (25-200 kDa, *e.g.,* from about 25 to about 200 kDa). In some embodiments, the molecular weight (e.g., medium range molecular weight) of the derivatized polyglucosamine is between 15 and 1,000 kDa. In some embodiments, the molecular weight (e.g., medium range molecular weight) of the derivatized polyglucosamine is between 20 and 450 kDa. In some embodiments, the molecular weight (e.g., medium range molecular weight) of the derivatized polyglucosamine is between 20

and 350 Da. In some embodiments, the molecular weight (e.g., medium range molecular weight) of the polyglucosamine derivative is between 25 and 200 kDa.

The functionalized polyglucosamine derivatives described herein include the following:
(A) Polyglucosamine-arginine compounds;
Other polyglucosamine derivatives in the present disclosure that are not encompassed in the claims are:
(B) Polyglucosamine-natural amino acid derivative compounds;
(C) Polyglucosamine-unnatural amino acid compounds;
(D) Polyglucosamine-acid amine compounds;
(E) Polyglucosamine-guanidine compounds; and
(F) Neutral polyglucosamine derivative compounds.

### (A) Polyglucosamine-arginine compounds

In some embodiments, the present invention is directed to polyglucosamine-arginine compounds for use in a method of treating dysbiosis, where the arginine is bound through a peptide (amide) bond via its carbonyl to the primary amine on the glucosamines of polyglucosamine: 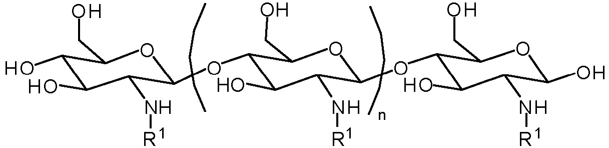 wherein each R¹ is independently selected from hydrogen, acetyl, and a group of the following formula: 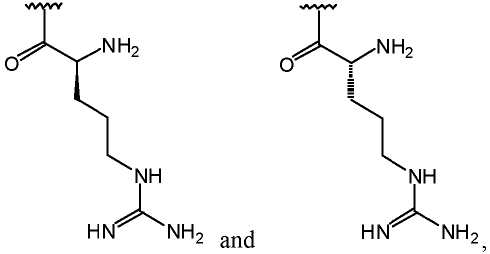 or a racemic mixture thereof,
wherein at least 25% of R¹ substituents are H, at least 1% are acetyl, and at least 2% are a group of the formula shown above. In some embodiments, a polyglucosamine-arginine compound is of the following formula 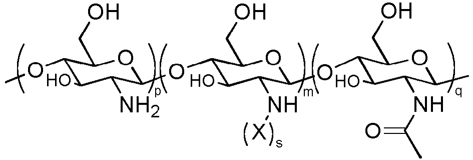 where m is 0.02-0.50; q is 0.50-0.01; s is 1; p+q+m= 1; the percent degree of functionalization is m • 100%; and X is selected from the group consisting of: 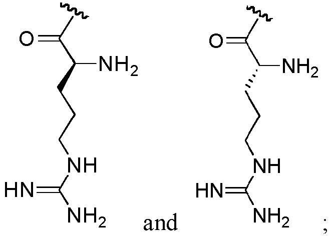 wherein the preparation is substantially free of compounds having a molecular weight of less than 5 kDa.

In some embodiments, the molecular weight average of polyglucosamine-arginine compound is (e.g., 20 to 200 kD, 20 to 150 kD, 30 to 120 kD, 50 to 100 kD) 25 to 80 kD. In some embodiments, the molecular weight range of polyglucosamine-arginine compound is 20 to 350 kD. In some embodiments, the molecular weight average of polyglucosamine-arginine compound is 25 to 125 kD. In some embodiments, the molecular weight average of polyglucosamine-arginine compound is 25 to 70 kD. In some embodiments, the molecular weight range of polyglucosamine-arginine compound is 100 to 300 kD.

In some embodiments, the molecular weight (e.g., weight average molecular weight) of polyglucosamine-arginine compound is from 20 to 350 kDa. In some embodiments, the molecular weight (e.g., weight average molecular weight) of polyglucosamine-arginine compound is from 50 to 120 kDa. In some embodiments, the molecular weight (e.g., weight average molecular weight) of polyglucosamine-arginine compound is from 25 to 80 kDa.

In some embodiments, the polyglucosamine-arginine compound is 25 to 35% functionalized.

In some embodiments, the polyglucosamine derivative is 10% to 45% functionalized. In some embodiments, the of polyglucosamine-arginine compound is arginine-functionalized at between 15% and 40%. In some embodiments, the of polyglucosamine-arginine compound is arginine-functionalized at between 20% and 40%. In some embodiments, the polyglucosamine-arginine compound is arginine-functionalized at between 20% and 30%. In some embodiments, the polyglucosamine-arginine compound is arginine-functionalized at between 25% and 35%. In some embodiments, the polyglucosamine-arginine compound is arginine-functionalized at least 18%. In some embodiments, the polyglucosamine-arginine compound is arginine-functionalized at about 18% to about 40%. In some embodiments, the polyglucosamine derivative is 18% to 35% functionalized.

### (B) Polyglucosamine-natural amino acid derivative compounds

In some aspects of the disclosure that are not encompassed by the claims the polyglucosamine derivative is directed to polyglucosamine-natural amino acid derivative compounds, wherein the natural amino acid may be histidine or lysine. The amino is bound through a peptide (amide) bond via its carbonyl to the primary amine on the glucosamines of polyglucosamine: 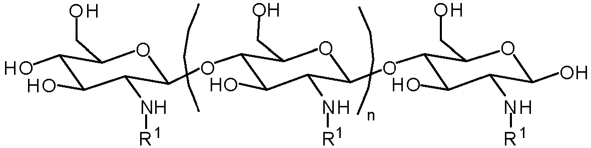 wherein each R¹ is independently selected from hydrogen, acetyl, and a group of the following formula: 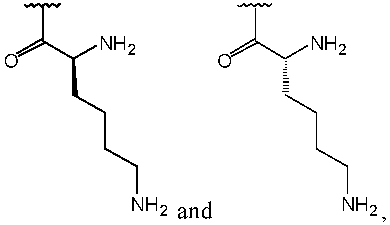 or a racemic mixture thereof, wherein at least 25% of R¹ substituents are H, at least 1% are acetyl, and at least 2% are a group of the formula shown above; or a group of the following formula: 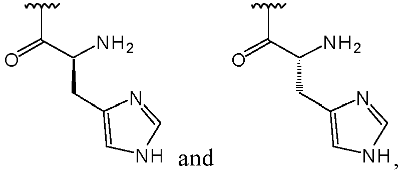 or a racemic mixture thereof, wherein at least 25% of R¹ substituents are H, at least 1% are acetyl, and at least 2% are a group of the formula shown above.

### (C) Polyglucosamine-unnatural amino acid compounds

In some aspects of the disclosure that are not encompassed by the claims the polyglucosamine derivative is directed to polyglucosamine-unnatural amino acid compounds, where the unnatural amino acid is bound through a peptide (amide) bond via its carbonyl to the primary amine on the glucosamines of polyglucosamine: 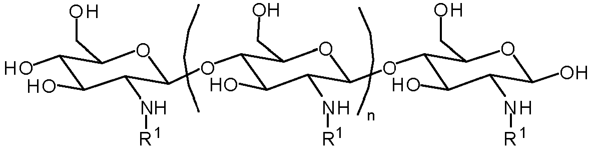 wherein each R¹ is independently selected from hydrogen, acetyl, and a group of the following formula: 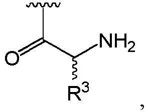 wherein R³ is an unnatural amino acid side chain, and wherein at least 25% of R¹ substituents are H, at least 1% are acetyl, and at least 2% are a group of the formula shown above.

Unnatural amino acids are those with side chains not normally found in biological systems, such as ornithine (2,5-diaminopentanoic acid). Any unnatural amino acid may be used in accordance with the invention. In some embodiments, the unnatural amino acids coupled to polyglucosamine have the following formulae:

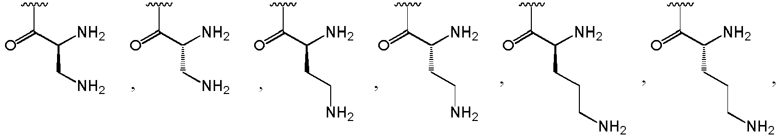

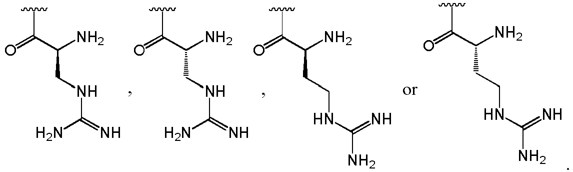

### (D) Polyglucosamine-acid amine compounds

In some aspects of the disclosure that are not encompassed by the claims the polyglucosamine derivative is directed to polyglucosamine-acid amine compounds, or their guanidylated counterparts. The acid amine is bound through a peptide (amide) bond via its carbonyl to the primary amine on the glucosamines of polyglucosamine: 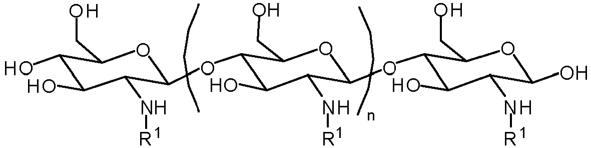 wherein each R¹ is independently selected from hydrogen, acetyl, and a group of the following formula: 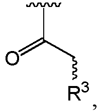 wherein R³ is selected from amino, guanidino, and C₁-C₆ alkyl substituted with an amino or a guanidino group, wherein at least 25% of R¹ substituents are H, at least 1% are acetyl, and at least 2% are a group of the formula shown above

In some aspects, R¹ is selected from one of the following:

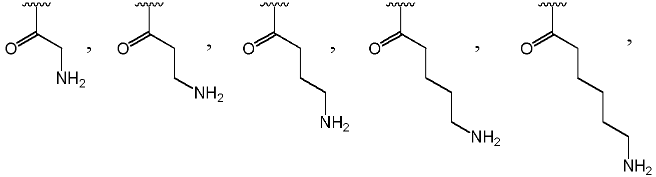

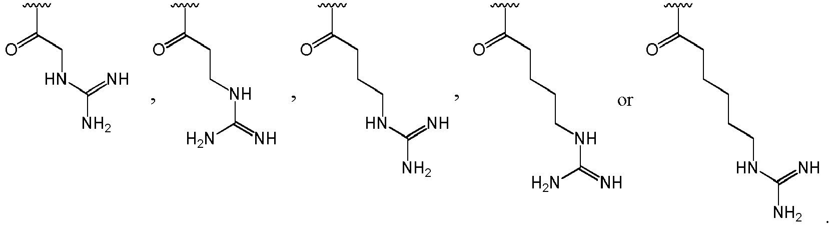

### (E) Polyglucosamine-guanidine compounds

In some aspects of the disclosure that are not encompassed by the claims the polyglucosamine derivative is directed to polyglucosamine-guanidine compounds. 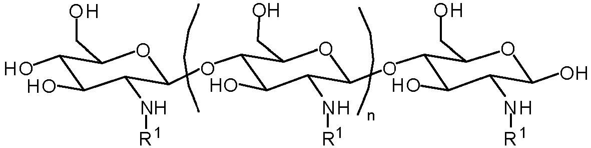 wherein each R¹ is independently selected from hydrogen, acetyl, and a group in which R¹, together with the nitrogen to which it is attached, forms a guanidine moiety; wherein at least 25% of R¹ substituents are H, at least 1% are acetyl, and at least 2% form a guanidine moiety together with the nitrogen to which it is attached.

### (F) Neutral polyglucosamine derivative compounds

In some aspects of the disclosure that are not encompassed by the claims the polyglucosamine derivative is directed to neutral polyglucosamine derivative compounds. Exemplary neutral polyglucosamine derivative compounds include those where one or more amine nitrogens of the polyglucosamine have been covalently attached to a neutral moiety such as a sugar: 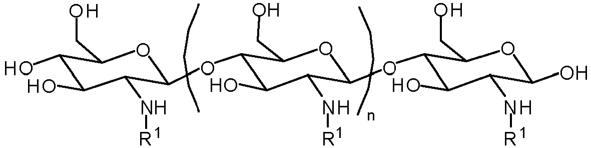 wherein each R¹ is independently selected from hydrogen, acetyl, and a sugar (*e.g.,* a naturally occurring or modified sugar) or an α-hydroxy acid. Sugars can be monosaccharides, disaccharides or polysaccharides such as glucose, mannose, lactose, maltose, cellubiose, sucrose, amylose, glycogen, cellulose, gluconate, or pyruvate. Sugars can be covalently attached via a spacer or via the carboxylic acid, ketone or aldehyde group of the terminal sugar. Examples of □-hydroxy acids include glycolic acid, lactic acid, and citric acid. In some preferred examples, the neutral polyglucosamine derivative is polyglucosamine-lactobionic acid compound or polyglucosamine-glycolic acid compound. Exemplary salts and coderivatives include those known in the art, for example, those described in US 8,119,780.

### Compositions and dosage forms

Described herein are methods for modulating or perturbing the microbiome environment with compositions comprising a polyglucosamine as described herein (e.g., polyglucosamine-arginine (PAAG)). In some embodiments, the composition described herein is configured for oral administration.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or carriers and processing aids helpful for forming the desired dosing form.

In some embodiments, the composition described herein is configured as a solid dosage formulation. For example, the composition can be a dry powder that is used in a capsule or tablet (e.g., compressed with cellulose). In some embodiments, the composition is a dry powder dissolved in water. In some embodiments, the compositions are configured for controlled release or timed release, e.g., in a gel capsule, in the gastrointestinal tract.

In some embodiments, the compositions are oven-dried, freeze-dried, or spray-dried.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring. In some embodiments, the compositions described herein is configured as a liquid formulation (e.g., aqueous formulation, e.g., aqueous formulation without stabilizer).

Liquid forms suitable for rectal administration, such as enemas, may include suitable aqueous or nonaqueous vehicles comprising buffers, suspending and dispensing agents, colorants, and the like. Exemplary excipients for enema formulations comprise sodium chloride, sodium bicarbonate, sodium monohydrogen phosphate, sodium dihydrogen phosphate, glycerin, docusate, mineral oil, ethanol, propylene glycol, and polyethylene glycol. Solid forms for rectal administration includes suppositories, which can prepared to melt or dissolve when inserted into the rectum. Exemplary excipients for suppository formulations include cocoa butter, propylene glycol, polyethylene glycol, and agar.

### Course of treatment

Described herein are a course of treatment for modulating or perturbing the microbiome environment with compositions comprising a polyglucosamine as described herein (e.g., polyglucosamine-arginine (PAAG)). In some embodiments, the composition is administered to the subject one to four times daily. In some embodiments, the composition is administered to the subject once or twice daily.

In some embodiments, 10 to 5000 mg (e.g., 50 to 250 mg, 250 to 1000 mg) of the composition is administered to the subject per dose or per day. In some embodiments, 0.1 µg to 1 mg (e.g., 1 µg to 1 mg, 0.1 µg to 500 µg, 1 µg to 500 µg) of the composition (e.g., the composition comprising a compound described herein) is administered to the subject per dose or per day.

In some embodiments, the composition is administered once daily (e.g., for 1, 2, 3, 4 weeks). In some embodiments, the composition (e.g., composition as described herein, e.g., composition comprising a compound described herein) is administered 1, 2, 3, or 4 times per day. In some embodiments, the composition (e.g., composition as described herein, e.g., composition comprising a compound described herein) is administered at 4 to 50 mg/kg/dose/day.

### Antibacterials

The methods described herein can be used to treat a subject as described herein (e.g., a subject with one or more diseases and conditions described herein). In some embodiments, the subject has been treated with an antibacterial or antibiotic (e.g., the subject has been previously treated with an antibacterial or antibiotic).

General classes of antibiotics include, e.g., aminoglycosides, bacitracin, beta-lactam antibiotics, cephalosporins, chloramphenicol, glycopeptides, macrolides, lincosamides, penicillins, quinolones, rifampin, glycopeptide, tetracyclines, trimethoprim and sulfonamides. Exemplary antibiotics within the classes recited above are provided as follows. Exemplary aminoglycosides include Streptomycin, Neomycin, Framycetin, Parpmycin, Ribostamycin, Kanamycin, Amikacin, Dibekacin, Tobramycin, Hygromycin B, Spectinomycin, Gentamicin, Netilmicin, Sisomicin, Isepamicin, Verdamicin, Amikin, Garamycin, Kantrex, Netromycin, Nebcin, and Humatin. Exemplary carbacephems include Loracarbef (Lorabid). Exemplary carbapenems include Ertapenem, Invanz, Doripenem, Finibax, Imipenem/Cilastatin, Primaxin, Meropenem, and Merrem. Exemplary cephalosporins include Cefadroxil, Durisef, Cefazolin, Ancef, Cefalotin, Cefalothin, Keflin, Cefalexin, Keflex, Cefaclor, Ceclor, Cefamandole, Mandole, Cefoxitin, Mefoxin, Cefprozill, Cefzil, Cefuroxime, Ceftin, Zinnat, Cefixime, Suprax, Cefdinir, Omnicef, Cefditoren, Spectracef, Cefoperazone, Cefobid, Cefotaxime, Claforan, Cefpodoxime, Fortaz, Ceftibuten, Cedax, Ceftizoxime, Ceftriaxone, Rocephin, Cefepime, Maxipime, and Ceftrobriprole. Exemplary glycopeptides include Dalbavancin, Oritavancin, Teicoplanin, Vancomycin, and Vancocin. Exemplary macrolides include Azithromycin, Sithromax, Sumamed, Zitrocin, Clarithromycin, Biaxin, Dirithromycin, Erythromycin, Erythocin, Erythroped, Roxithromycin, Troleandomycin, Telithromycin, Ketek, and Spectinomycin. Exemplary monobactams include Aztreonam. Exemplary penicillins include Amoxicillin, Novamox, Aoxil, Ampicillin, Alocillin, Carbenicillin, Coxacillin, Diloxacillin, Flucloxacillin Floxapen, Mezlocillin, Methicillin, Nafcillin, Oxacillin, Penicillin, and Ticarcillin. Exemplary polypeptides include Bacitracin, Colistin, and Polymyxin B. Exemplary quiniolones include Ciproflaxin, Cipro, Ciproxin, Ciprobay, Enoxacin, Gatifloxacin, Tequin, Levofloxacin, Levaquin, Lomefloxacin, Moxifloxacin, Avelox, Norfloxacin, Noroxin, Ofloxacin, Ocuflox, Trovafloxacin, and Trovan. Exemplary sulfonamides include Mefenide, Prontosil (archaic), Sulfacetamide, Sulfamethizole, Sulfanilamide (archaic), Sulfasalazine, Sulfisoxazole, Trimethoprim, Trimethoprim-Sulfamethoxazole (co-trimoxazole), and Bactrim. Exemplary tetracyclines include Demeclocyline, Doxycycline, Vibramycin, Minocycline, Minocin, Oxytetracycline, Terracin, Tetracycline, and Sumycin. Other exemplary antibiotics include Azlocillin, Bacampillicin, Salvarsan, Clavulanic acid, Chloamphenicol, Chloromycetin, Clindamycin, Cleocin, Cefaloridine, Cefbuperazone, Cefmenoxime, Cefotetan, Ceftazadine, Cephardine, Cephrocile, Linomycin, Ethambutol, Fosfomycin, Fusidic Acid, Fucidin, Furazolidone, Isoniazid, Linezolid, Zyvox, Metronidazole, Flagyl, Mupirocin, Bactroban, Nitrofurantion, Macrodantin, Macrobid, Moxalactam, Piperacillin, Pivampicillin, Pivmecillinam, Phenoxymethylpenicillin, Pefloxacin, Platensimycin, Pyrazinamide, Quinupristin/Dalfopristin (Syncerid), Rifampin (Rifampicin), Sulbactam, Talampicillin, Temocillin, and Tinidazole.

### Anti-inflammatory

The compositions and compounds described herein (e.g., soluble polyglucosamines and derivatized polyglucosamines) can be used in combination with or sequentially with (e.g., in series with, before, or after) one or more anti-inflammatory drugs, e.g., steroidal anti-inflammatory drugs and non-steroidal anti-inflammatory drugs (NSAIDs), to treat one or more diseases or conditions described herein. In some embodiments, the administrations of a combination of agents and therapeutics are spaced sufficiently close together such that a synergistic effect is achieved.

Exemplary steroidal anti-inflammatory drugs include glucocorticoids (corticosteroids), e.g., Hydrocortisone (Cortisol), Cortisone acetate, Prednisone, Prednisolone, Methylprednisolone, Dexamethasone, Betamethasone, Triamcinolone, Beclometasone, Fludrocortisone acetate, Deoxycorticosterone acetate (DOCA), and Aldosterone. Exemplary non-steroidal anti-inflammatory drugs include Aspirin, Choline and magnesium salicylates, Choline salicylate, Celecoxib, Diclofenac potassium, Diclofenac sodium, Diclofenac sodium with Isoprostol, Diflunisal, Etodolac, Fenoprofen calcium, Flurbiprofen, Ibuprofen, Indomethacin, Ketoprofen, Magnesium salicylate, Meclofenamate sodium, Mefenamic acid, Meloxicam, Nabumetone, Naproxen, Naproxen sodium, Oxaprozin, Piroxicam, Rofecoxib, Salsalate, Sodium salicylate, Sulindac, Tolmetin sodium, and Valdecoxib. Exemplary non-steroidal anti-inflammatory agents (e.g., peptides) include regulatory cytokines, such as interleukins, e.g., IL-1, IL-4, IL-6, IL-10, IL-11, and IL-13.

### Subject

The subject can be a human or an animal. Suitable animal subjects include primates, mammals, rodents, and birds. In some embodiments, the subject is a human subject. In some embodiments, the human subject is from 1 to 50 year(s) old (e.g., from 18 to 42 years old). In some embodiments, the human subject is a child (e.g., older child) or adult (e.g., young adult). In some embodiments, the human subject is from 1 to 35 year(s) old. In some examples, the human subject has cystic fibrosis.

In some examples, the subject has diseases or conditions characterized by the presence of one or more of the bacteria that cause resistant bacterial infection as described herein. In some examples, the subject has higher levels of a bacteria (e.g., bacteria as described herein) relative to a reference standard. In some examples, the subject has been exposed to radiation (e.g., the subject is identified as a subject who has been exposed to radiation). In some embodiments, the subject has been previously been administered an antibiotic (e.g., an antibacterial as described herein) (e.g., the subject is identified as a subject who has been administered an antibiotic (e.g., an antibacterial as described herein). In some examples, the subject is identified as a subject who has consumed a diet high in sulfates or proteins. In some examples, the subject has consumed (e.g., a high amount of) alcohol (e.g., a subject identified to have consumed more than 1, 2, or 3 pints of alcohol per day or per week).

In some examples, the subject has a bacterial infection (e.g., a bacterial infection as described herein). In some examples, the bacterial infection is *Salmonella, Campylobacter, S. Typhimurium,* or *C. jejuni* infection. In some examples, the bacterial infection is *Pseudomonas aeruginosa,* methichillin resistant *Staphylococcus aureus, Acinetobacter baumannii,* or *Burkholderia cepacia* infection. In some examples, the bacterial infection is *Clostridium perfringens* infection. In some examples, the bacterial infection comprises a species of *Campylobacter* (e.g., *Campylobacter jejuni*), *Escherichia* (*Escherichia coli*), *Salmonella, Shigella,* or *Staphylococcus* (*Staphylococcus aureus*).

In some embodiments, the subject has been treated previously with an antibacterial or antibiotic agent.

### Materials and Methods

The compounds provided herein can be prepared from readily available starting materials using the following general methods and procedures. It will be appreciated that where typical or preferred process conditions (*i.e.,* reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

### Examples

In order that the invention described herein may be more fully understood, the following examples are set forth. The synthetic and biological examples described in this application are offered to illustrate the compounds, pharmaceutical compositions and methods provided herein and are not to be construed in any way as limiting their scope. As used in herein (e.g., the **Examples** and **Figures**), TNBS refers to 2,4,6-trinitrobenzenesulfonic acid; chitosan-arginine and PAAG refers to polyglucosamine-arginine.

### Comparative Example 1. Reduction of the mean endoscopy proctitis scores in the radiation proctitis model following twice daily rectal treatment with PAAG.

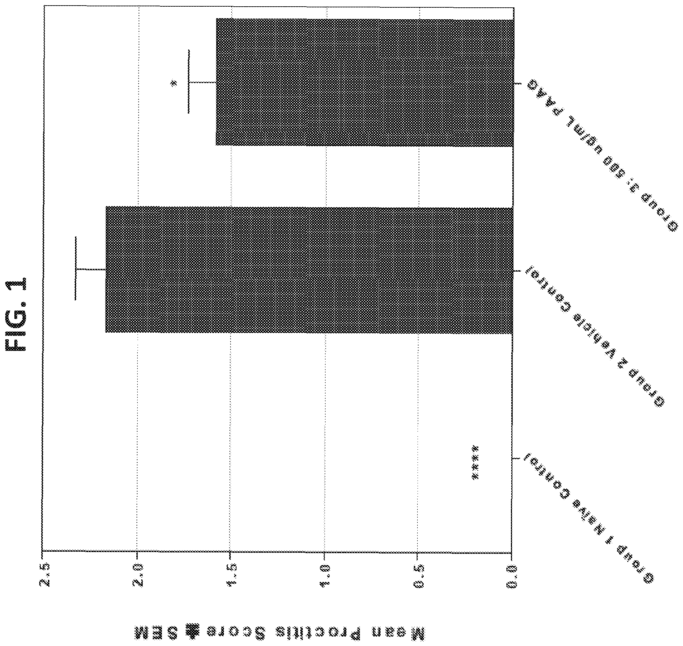

A twice daily 500 ug/mL PAAG (61kD, 28% functionalization) rectal enema is sufficient to reduce proctitis in a rat model. In this model, proctitis was induced in 16 male rats (per group) with a single 20 Gy exposure on Day 0. Lead shielding covered the rats except for a 3cm X 4cm area of the low pelvis. This area contains approximately a 2cm length of the rectum in the middle of the field. The rats were treated for 7 days, twice daily with 500 µg/mL PAAG or vehicle control rectally, starting at day -1. Animals underwent video endoscopy to evaluate disease severity at day 7 (**FIG. 1**). Proctitis severity was scored on a scale of 0-4. A score of 0 for normal, 1 for loss of vascularity, 2 for loss of vascularity and friability, 3 for friability and erosions, and 4 for severe ulcerations and bleeding. The data in **FIG. 1** represent group means ± SEM. Statistically significant differences among the treatment group (Group 3) in comparison to the vehicle control group (Group 2) were evaluated using One-Way ANOVA followed by Holm-Sidak's multiple comparisons test in comparison to the vehicle control group. The PAAG 500µg/mL enema group had observable differences in inflammation, erythema and necrosis relative to vehicle control, and was statistically superior to the vehicle control (*p<0.05). **FIG. 1** shows twice daily rectal PAAG treatment significantly reduced proctitis in a rat model.

### Comparative Example 2. Reduction of local and systemic biomarkers for inflammation in the radiation proctitis model following twice daily rectal treatment with PAAG.

It has been demonstrated that procalcitonin (PCT) is a biomarker of systemic inflammation in mice for the acute gastrointestinal radiation syndrome and markers of infection in neutropenic adults. Calprotectin is a known fecal marker of local inflammation and comprises up to 60% of the soluble protein content in neutrophils, key cellular components of inflammation. In inflammatory conditions with neutrophil influx, calprotectin has been used successfully as a fecal marker of gastrointestinal inflammation. Calprotectin is stable in the feces and a non-invasive biomarker of inflammatory bowel diseases.

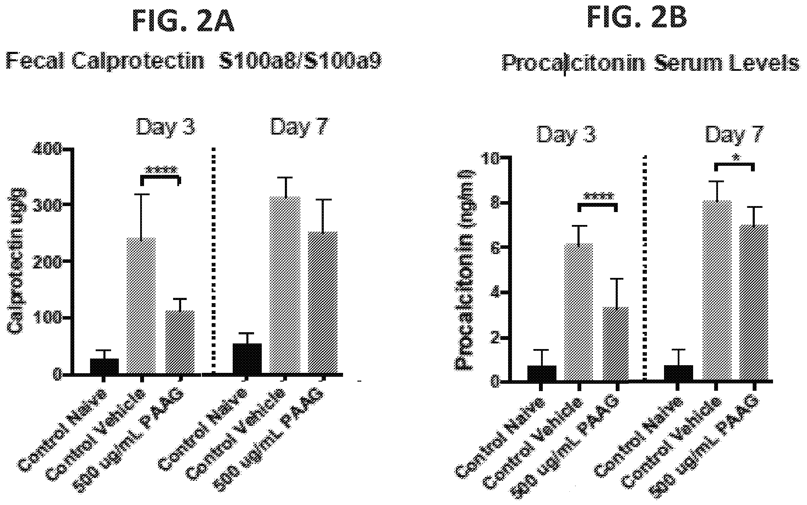

A twice daily 500 ug/mL PAAG (61kD, 28% functionalization) rectal enema is sufficient to reduce systemic and local inflammation in a rat proctitis model. Briefly, proctitis was induced in 16 male rats (per group) with a single 20 Gy exposure on Day 0. Lead shielding covered the rats except for a 3cm X 4cm area of the low pelvis. This area contains approximately a 2cm length of the rectum in the middle of the field. The rats were treated for 7 days, twice daily with 500 µg/mL PAAG or vehicle control rectally, starting at day -1. Blood and feces were collected at day 3 and 7 (**FIGS. 2A-2B**).

Frozen serum samples were processed for procalcitonin concentration. The data was expressed in ng/ml of procalcitonin in the serum. Rats that were not exposed to radiation (n=5) showed low levels of calprotectin in the feces at both Day 3 and 7, as expected. **FIG. 2B** shows a significant difference in the concentration of procalcitonin was observed between the vehicle treated rats and rats treated with 500 ug/ml of PAAG. On Day 3 specifically, untreated rats had 6.1 ng/ml procalcitonin in serum samples compared to rats treated with 500 PAAG that had 3.2 (p<0.0001) of serum procalcitonin (p<0.001). On Day 7 the observation is also observed between the vehicle and rats treated with 500 ug/ml PAAG (p<0.05).

Frozen fecal samples were processed for calprotetin concentration. The data was expressed in ug of calprotetin per gram of tissue. Rats that were not exposed to radiation showed low levels of calprotectin in the feces as expected. **FIG. 2A** shows a significant difference in the concentration of calprotectin was observed on Day 3 between the vehicle treated rats and rats treated with 500 ug/ml of PAAG rectally, twice daily (p<0.0001). **FIGS. 2A-2B** show twice daily rectal PAAG treatment significantly reduced systemic and local gastrointestinal inflammation in a rat model.

### Comparative Example 3. Reduction of local and systemic biomarkers for inflammation in the radiation proctitis model following daily oral treatment with PAAG.

It has been demonstrated that procalcitonin (PCT) is a biomarker of systemic inflammation in mice for the acute gastrointestinal radiation syndrome and markers of infection in neutropenic adults. Calprotectin is a known fecal marker of local inflammation and comprises up to 60% of the soluble protein content in neutrophils, key cellular components of inflammation. In inflammatory conditions with neutrophil influx, calprotectin has been used successfully as a fecal marker of gastrointestinal inflammation. Calprotectin is stable in the feces and a non-invasive biomarker of inflammatory bowel diseases.

A daily 50 mg/kg oral PAAG (61kD, 28% functionalization) treatment is sufficient to reduce proctitis in a rat model. In this model, proctitis was induced with a single 20 Gy exposure on Day 0. Lead shielding covered the rats except for a 3cm X 4cm area of the low pelvis. This area contains approximately a 2cm length of the rectum in the middle of the field. Oral PAAG (50 mg/kg or vehicle control) delivered from Day -3 to 5, twice daily. Feces and blood collected every other day.

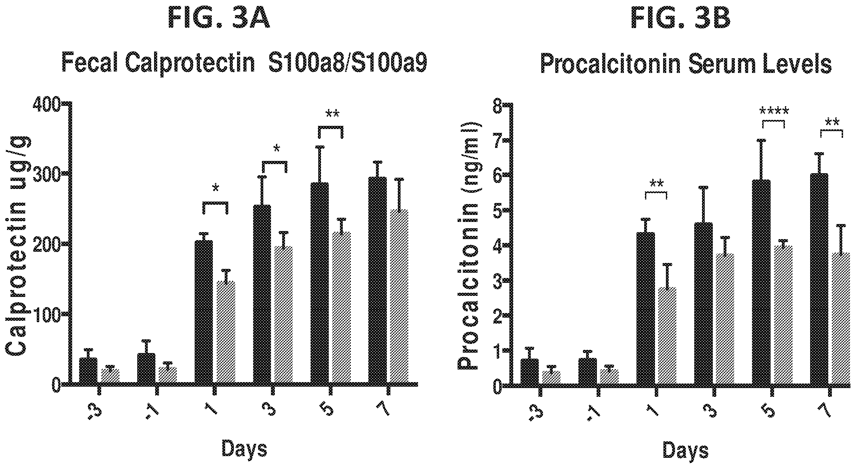

Frozen serum samples were processed for procalcitonin concentration. The data was expressed in ng/ml of procalcitonin in the serum. Rats that were not exposed to radiation (n=5) showed low levels of calprotectin in the feces at both Day 3 and 7, as expected. **FIG. 3B** shows a significant difference in the concentration of procalcitonin was observed between the vehicle treated rats and rats treated with 50 mg/kg of PAAG daily. On Day 1, 5, and 7 specifically, untreated rats had significantly more procalcitonin in serum samples compared to rats treated with 50 mg/kg of PAAG daily (p<0.01). PAAG reduces the amount of systemic inflammation as reflected by the smaller amount of plasma procalcitonin.

Frozen fecal samples were processed for analysis. The data was expressed in ug of calprotetin per gram of tissue. Rats that were not exposed to radiation showed low levels of calprotectin in the feces as expected. **FIG. 3A** shows a significant difference in the concentration of calprotectin was observed on Days 1, 3, and 5 between the vehicle treated rats and rats treated with 50 mg/kg of PAAG daily (p<0.05). **FIGS. 3A-3B** shows daily oral PAAG treatment significantly reduced systemic and local gastrointestinal inflammation in a rat model.

### Example 4. Reduction of necrotic enteritis in a model of Clostridium infection by treatment with PAAG

*Clostridium perfringens* (CP) is the poultry equivalent of *C. difficile* in humans, causing recurrent Gi infection, GI inflammation and often high mortality. In order to assess the efficacy of PAAG in treating the inflammation and infection in *Clostridum* infections, a poultry study of infection was completed. Nine pens of 10 one-day-old male broiler chicks were purchased from Cobb-Vantress hatchery, Cleveland, GA. Six hundred and thirty chicks were assigned to this study (90 per arm). The strain was Cobb X Cobb. Breeder flock number was F-4420 and was 47 weeks old at time of lay. Feed and water were available *ad libitum* throughout the trial. On DOT 0, all birds (except controls), were orally inoculated with a coccidial inoculum containing approximately 5,000 oocysts of *E. maxima* per bird. On DOT 5, all birds, except controls, were given a broth culture of *C. perfringens* approximately 10⁸ cfu/ml Various doses of PAAG or bacitracin (BMD, positive control) or water (negative control) were given via continuous source in water at 1 day prior to CP infection and for 5 days post infection. PAAG used in this study has an average molecular weight of 22kDa, 36% functionalized, %DDA= 89 and PDI=1.7. On DOT 7, three birds from each cage were selected, sacrificed, weighed, and examined for the degree of presence of Necrotic Enteritis lesions. The scoring was based on a 0 to 3 score, with 0 being normal and 3 being the most severe.

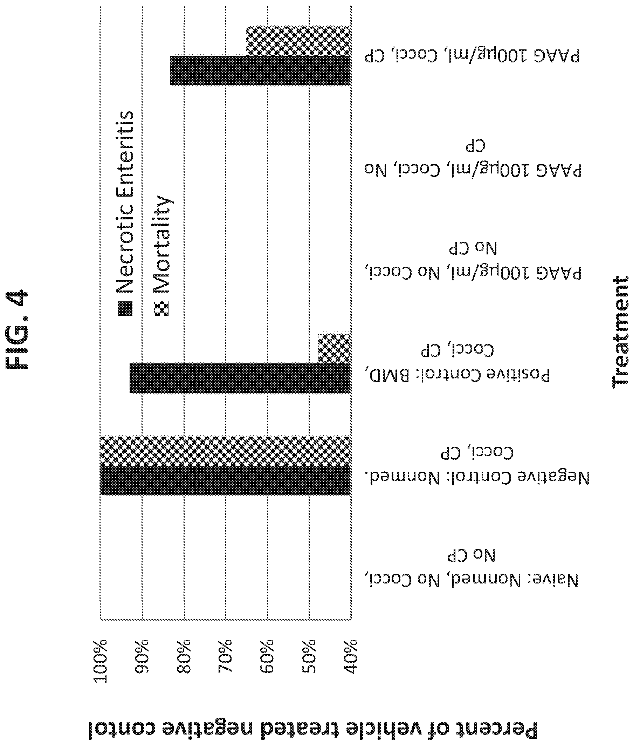

Data in **FIG. 4** compares positive control (BMD) and PAAG to vehicle treated animals (water only). As observed, PAAG does not irritate uninfected GI tract with either coccidia infection or coccidia and *C. perfringens.* (no inflammation, no mortality). PAAG reduces necrotic enteritis (NE) of the GI tract better than BMD (93%, P <0.05). PAAG treatment reduces NE by 83% (P<0.01) compared to vehicle treated animals.

### Comparative Example 5. Reduction of mortality in a model of Clostridium infection by treatment with PAAG

In the same experiment of **Example 4,** mortality of animals was assessed as a total aggregate deaths up to and including day 14. **FIG. 4** shows that PAAG reduces mortality from normalized untreated (100%) to 68% (P <0.05). BMD positive control reduces mortality to 45%. (P <0.001). PAAG given in the drinking water at 100µg/ml was sufficient to reduce mortality 42% from the observed mortality rate.

### Comparative Example 6. Differential gene expression in mice

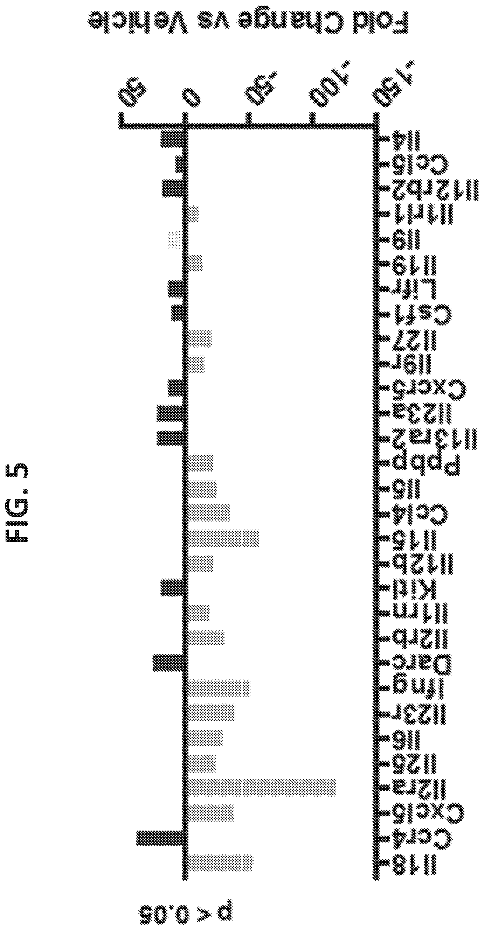

**FIG. 5** depicts mouse ileal tissue differential gene expression changes (red up regulated; green down regulated) in total body irradiated mice (30 Gy) at 4 days after irradiation and following treatment with 50 mg/kg PAAG daily starting 1 day after radiation relative to animals treated with vehicle control.

### Comparative Example 7. GI obstruction prevented by PAAG therapy in mice

**FIGS. 6A-6F** depict GI obstruction prevented by PAAG therapy in mice. **FIG. 6A****:** Adult CFTR^{-/-} mice were given PAAG (40 mg/kg/d) by oral gavage once daily for 21 days immediately after transition to a regular diet. P=0.08, n=6/condition. **FIG. 6B****:** Mice at weaning age were given PAAG (40 mg/kg/d) by oral gavage divided three times daily for 21 days while initiated on a regular diet. Kaplan-Meier survival curves for adult mice. P < 0.05, n=8/condition. **FIGS. 6C-6D****:** Representative images of gross intestine of control (**FIG. 6C**) and PAAG treated (**FIG. 6D**) mice. Arrow demonstrates distal-most site of obstruction. **FIGS. 6E-6F****:** PAS staining (10X) of control (FIG. 6**E**) and PAAG treated (**FIG. 6F**) intestine showing significant improvement of mucus impaction with PAAG treatment.

### Comparative Example 8. Biomarker levels after radiation inducing proctitis

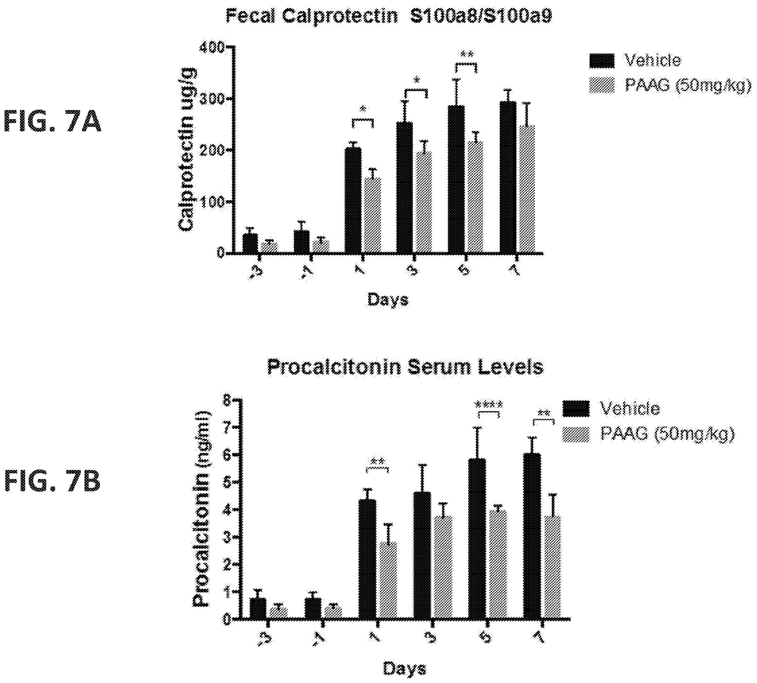

**FIGS. 7A-7B** depicts fecal calprotectin and serum procalcitonin measured in mice subjected to acute radiation on day 0 inducing proctitis. Serum and fecal samples were taken every other day prior to and after PAAG (50mg/ml daily) or vehicle control treatment. Both fecal calprotectin and procalcitonin were reduced in PAAG treated animals relative to vehicle control treated animals. * P≤0.05, **P≤0.01, ****P≤0.0001

### Example 9. Efficacy Study of PAAG in the Prevention of TNBS-Induced Colitis in Male Mice

The effect of oral dosing of PAAG was assessed on the colitis severity in the TNBS-induced model of colitis in male C57Bl/6 mice. Animals were dosed with test article or vehicle three times a day (t.i.d.) at 0.10 mL per dose, from day -1 to day 5 via oral gavage (p.o.). TNBS was administered via intrarectal administration of 100 µL of TNBS (4 mg) on day 0. Mice were examined on day 3 and 5 using video endoscopy to assess colitis severity. Upon sacrifice on day 5, serum was collected and colons were removed, measured, weighed, and fixed in 10% formalin for histological analysis. Weight loss occurred in all groups, with an average weight loss of at least 15.9% of starting weight in all groups by day 2, however the groups treated with PAAG regained weight at a faster rate than those animals treated with vehicle or prednisolone. Video endoscopy results on day 3 and day 5 revealed significant improvements in the mean colitis scores in both the prednisolone-treated group and the group treated with either 4 or 40 mg/kg t.i.d. PAAG when compared to the vehicle control group. There were no significant differences between treatment groups in either colon weight or colon length. Histopathology analysis of hematoxylin and eosin stained colon sections revealed significant decreases in colitis severity, as measured by inflammation, edema, and necrosis, in those groups treated with PAAG as well as in the group treated with prednisolone when compared to the vehicle treated control group.

### Experimental Design

Forty (40) male C57Bl/6 mice with average starting body weight of 25.6 g were were acclimatized for 5 days prior to study commencement. Colitis was induced in forty (40) male mice by intrarectal administration of 100 µL of TNBS (4 mg) in 50% ethanol under isoflurane anesthesia on day 0. Animals were dosed with test article or vehicle three times a day (t.i.d.) at 0.1 mL per dose, from day -1 to day 5 via oral gavage (p.o.). All animals were weighed daily and assessed visually for the presence of diarrhea and/or bloody stool. On day 3 and again on day 5 colitis severity was assessed in all animals using video endoscopy, where images were taken and colitis severity scored by a blinded observer. Following endoscopy on day 5, animals were sacrificed and the colon removed and its length and weight measured. The colon was dissected upon sacrifice. Colon length and weight were measured before the colon was fixed in 10% formalin. Cross-sections were obtained from the samples and stained with hematoxylin and eosin for histological examination. Statistical differences between a test group and the vehicle control were determined using a Student's t-test (SigmaPlot 11.2, Systat Software, Inc.).

Serum samples were obtained and the colon was fixed in 10% formalin. The details of the study design are shown in **Table 1.**

**Table 1. Study Design**

| **Group Number** | **Numbe r of Animal s** | **Treatment** | **Rout e** | **Dosing Schedule** | **Video Endoscopy Schedule** | **Amount Administered** |
|---|---|---|---|---|---|---|
| 1 | 10 males | Vehicle + TNBS | p.o. | t.i.d., days -1 to 5 | Day 3 & 5 | 0.10 mL |
| 2 | 10 males | Prednisolone 1 mg/kg/dose + TNBS | p.o. | t.i.d., days -1 to 5 | Day 3 & 5 | 0.10 mL |
| 3 | 10 males | PAAG 4 mg/kg/dose + TNBS | p.o. | t.i.d., days -1 to 5 | Day 3 & 5 | 0.10 mL |
| 4 | 10 males | PAAG 40 mg/kg/dose + TNBS | p.o. | t.i.d., days -1 to 5 | Day 3 & 5 | 0.10 mL |

Study endpoints were endoscopy colitis score, body weight change, survival, colon length, and colon weight. The presence of diarrhea and/or the presence of blood in the stool were assessed via visual assessment at the time of dosing. Animals were weighed daily throughout the study. Endoscopy was performed to evaluate colitis severity. Colitis was scored visually on a scale that ranges from 0 for normal, to 4 for severe ulceration. In descriptive terms, this scale is defined as follows:

**Table 2: Endoscopy Colitis Scoring Scale**

| **Score:** | **Description:** |
|---|---|
| 0 | Normal |
| 1 | Loss of vascularity |
| 2 | Loss of vascularity and friability |
| 3 | Friability and erosions |
| 4 | Ulcerations and bleeding |

To histologically evaluate colitis severity, a board certified veterinary pathologist with particular expertise in GI pathology evaluated colon tissue sections in a blinded fashion using a scale defined as depicted in Table 3. Five micron sections from each of four areas spanning the lower 5 cm of the colon were scored for each parameter. These scores were then averaged to obtain a single mean score per mouse per parameter.

**Table 3: Histology Colitis Scoring Scale**

| **Inflammation** | |
|---|---|
| **Score** | **Description** |
| 0 | None present |
| 1 | Rare foci; minimal |
| 2 | Scattered aggregates or mild diffuse inflammation |
| 3 | Numerous aggregates or moderate diffuse inflammation |
| 4 | Marked diffuse inflammation |

| **Edema** | |
|---|---|
| **Score** | **Description** |
| 0 | None present |
| 1 | Rare foci; minimal |
| 2 | Scattered regions or mild diffuse edema |
| 3 | Numerous regions or moderate diffuse edema |
| 4 | Marked diffuse edema |

| **Mucosal Necrosis** | |
|---|---|
| **Score** | **Description** |
| 0 | None present |
| 1 | <25% of the mucosa affected |
| 2 | 26-50% of the mucosa affected |
| 3 | 51-75% of the mucosa affected |
| 4 | >76% of the mucosa affected |

### Results

### Weight Change

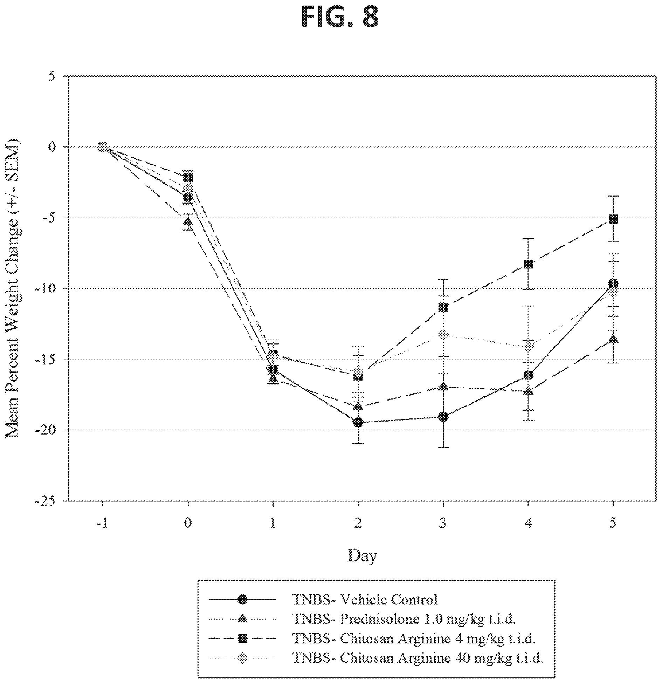

The mean daily percent weight gains for all treatment groups are shown in **FIG. 8****.** All groups of animals undergoing induction of colitis with TNBS exhibited at least 15.9% average weight loss by day 2. Average peak weight loss was observed on day 2 for all groups. Specifically, on day 2, the TNBS-treated vehicle control group had lost the most weight on average, with 19.5% of their starting weight. The prednisolone group lost an average of 18.3% loss from starting weight on day 2. Groups treated with PAAG lost an average of 11.3% (4 mg/kg t.i.d group) and 13.3% (40 mg/kg t.i.d. group) of their starting weight by day 2.

### Endoscopy- Colitis Scores

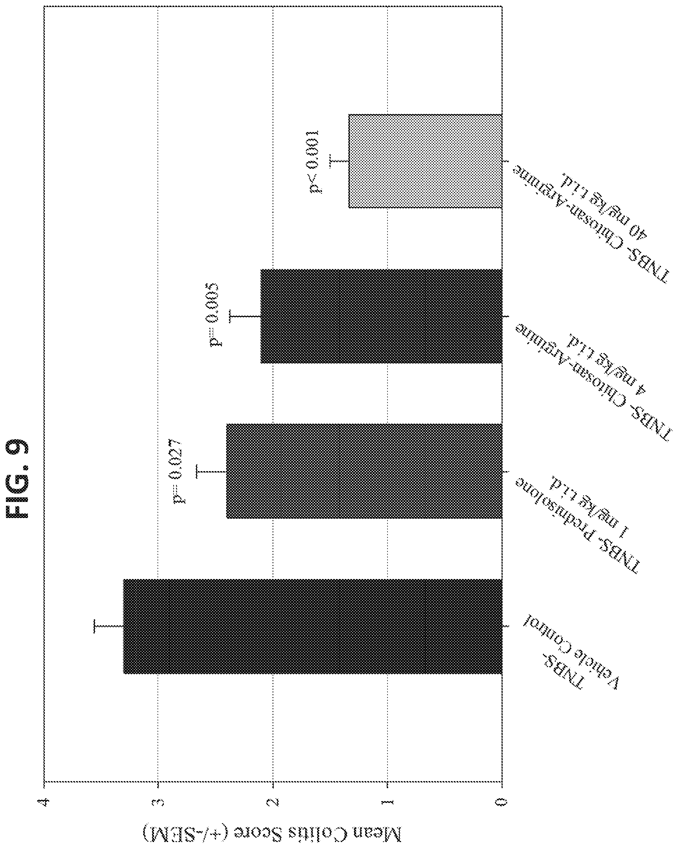

All animals underwent video endoscopy on day 3 and 5 to assess the severity of colitis in each treatment group. The mean colitis scores for all treatment groups are shown **FIG. 9** for day 5. Prednisolone treatment significantly reduced mean colitis scores on both day 3 (p= 0.010) and 5 (p= 0.027) as did PAAG at day 3 (P <0.001) when compared to the vehicle control group. Treatment with either 4 or 40 mg/kg doses of PAAG significantly reduced mean colitis scores on both days 3 and 5.

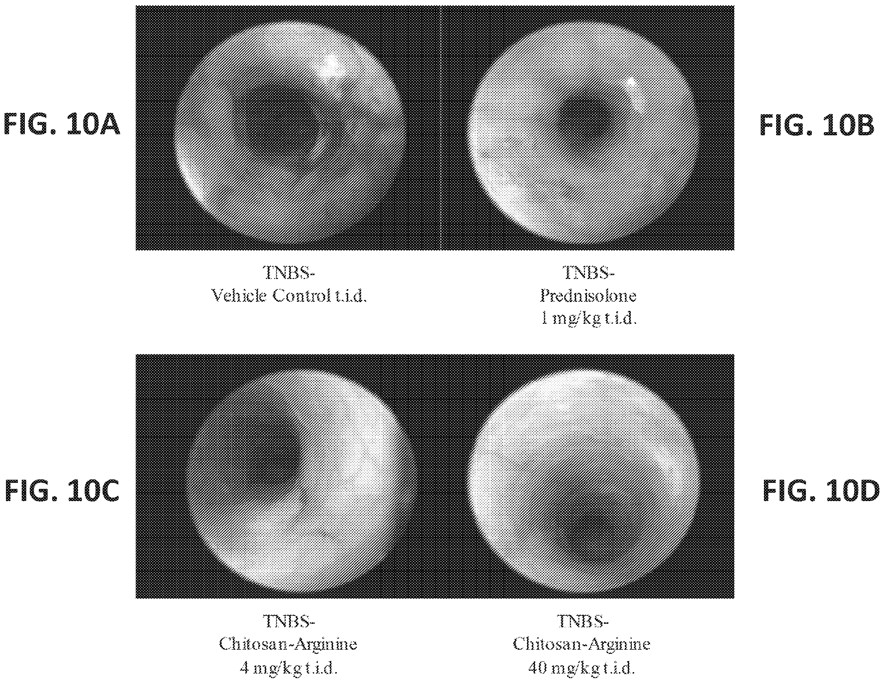

The response of the colon to the different treatment paradigms is apparent in the images presented in **FIGS. 10A-10D** that were captured via endoscopy on day 5. The corresponding colitis scores for the images presented in **FIGS. 10A-10D** are as follows: Vehicle control- score of 3, erosions, active bleeding, friability, and altered vascularity; Prednisolone - score of 2, friability and altered vascularity; PAAG 4 mg/kg t.i.d. - score of 2, friability and altered vascularity; PAAG 40 mg/kg t.i.d. - score of 1, altered vascularity.

### Pathology

Following endoscopy on day 5, colon tissue was removed, fixed in formalin, embedded in paraffin, and sectioned at approximately 5 microns. One slide for each colon (with one transverse section from each of 4 different areas of the colon per slide) was stained with hematoxylin and eosin (H&E) and examined by a board-certified veterinary pathologist. Tissues were scored for inflammation, edema, and mucosal necrosis according to the scoring criteria listed above in **Table 3.** Each of the 4 transverse sections was scored for these 3 parameters and the mean was reported for each animal for each parameter along with the mean sum score which was simply the sum of the three individual parameter scores. Due to the multifocal distribution of inflammation, edema, and mucosal necrosis, scores were variable among the transverse sections assessed for each colon sample.

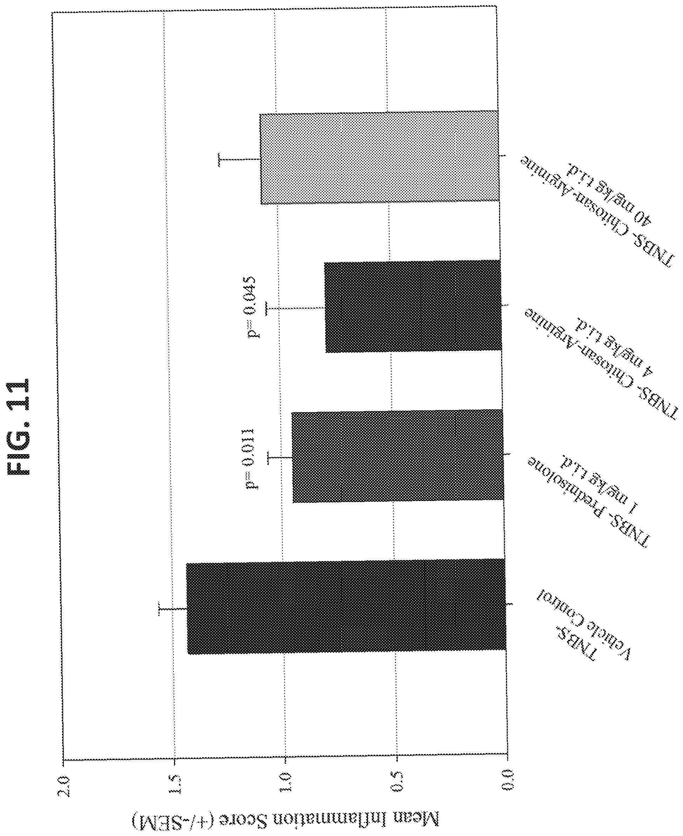

Inflammation scores (**FIG. 11**), defined as an infiltrate of any kind of inflammatory cell in an area where these cells would not normally be present, significantly differed among treatment groups. Both the prednisolone and 4 mg/kg t.i.d. PAAG groups displayed average inflammation scores under 1 which was significantly less than the vehicle control group (p= 0.011 and p= 0.045 respectively). No significant difference in inflammation scores was observed between the 40 mg/kg t.i.d. PAAG and vehicle control treated groups.

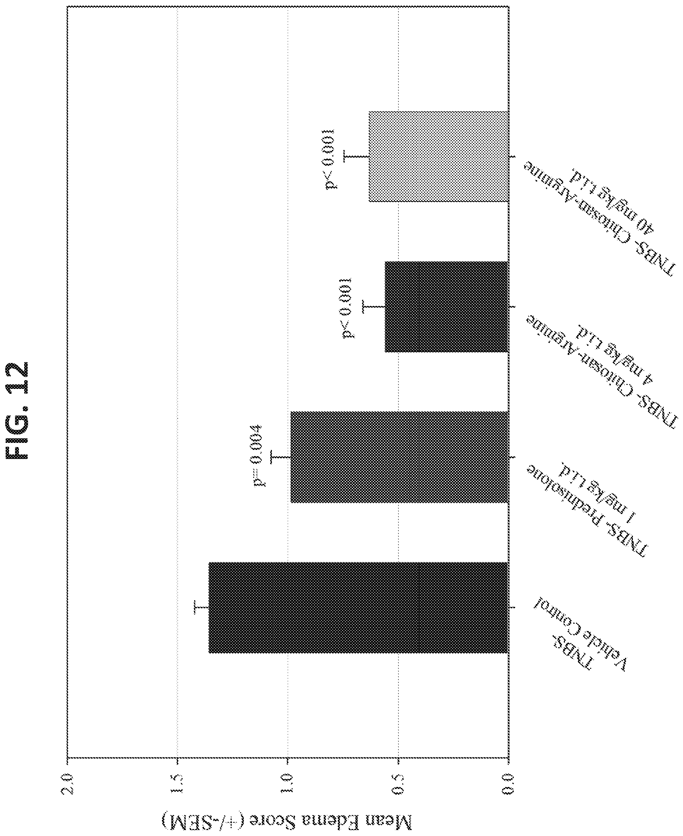

Edema scores (**FIG. 12**) were significantly lower in the prednisolone (p= 0.004) and PAAG (each dose p< 0.001) treatment groups as compared to the vehicle control group. All treatment groups had a mean edema score below 1, where a score of 1 represents rare foci or minimal edema.

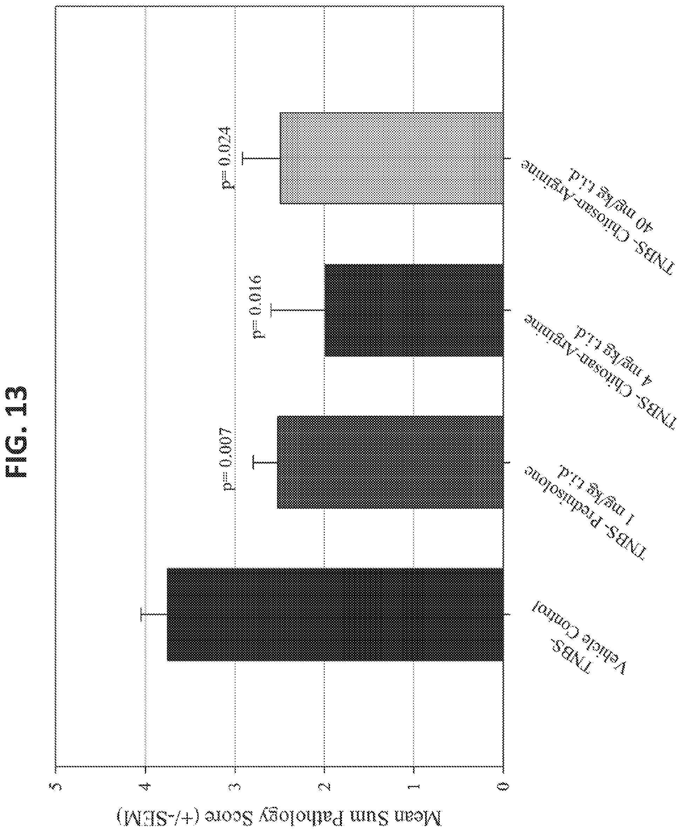

Sum pathology scores (**FIG. 13**), which take into account inflammation, edema, and mucosal necrosis, were significantly lower in the prednisolone (p= 0.007) and PAAG (p= 0.016 for the 4 mg/kg t.i.d. group; p=0.024 for the 40 mg/kg t.i.d. group) treatment groups as compared to the vehicle control group.

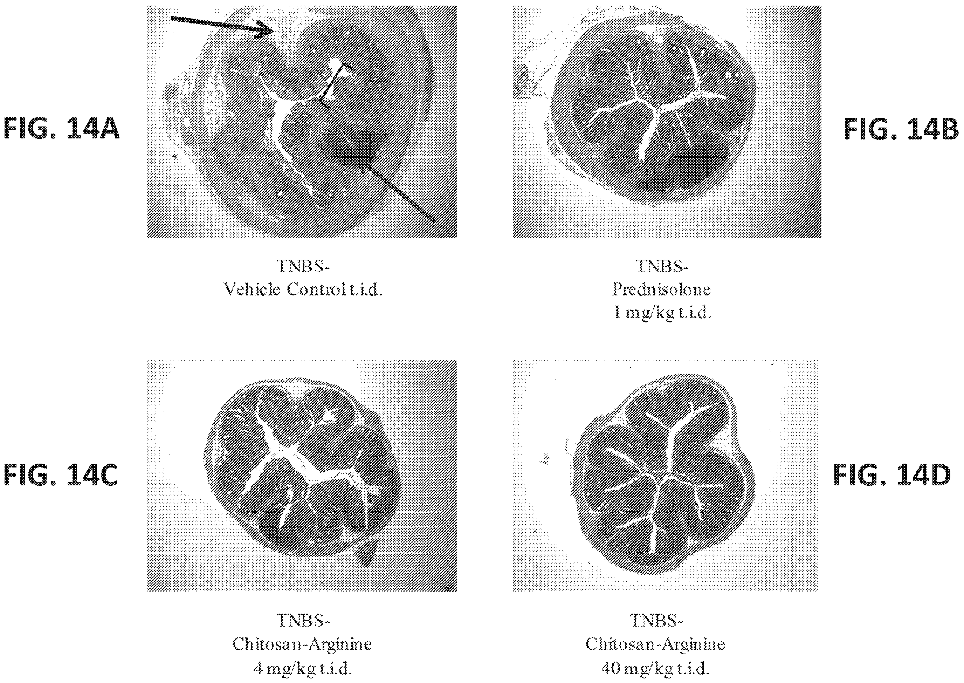

Representative photomicrographs for each treatment group are shown in **FIGS. 14A-14D****.** These images reveal that sections of colon in TNBS-treated animals are often thickened by inflammation and edema that variably extends into the lamina propria, submucosa, and muscular wall. The inflammation was primarily composed of neutrophils with lesser numbers of macrophages. Mucosal necrosis was also variably present and characterized by partial or complete loss of the surface epithelium and crypts. This mucosal necrosis often affected approximately 25% of the circumferential mucosal surface in the TNBS-treated control group. Due to the multifocal distribution of these changes, the inflammation, edema, and mucosal necrosis were variable among the 4 transverse sections assessed for each colon sample. The variability of the disease in the tissue sections is a likely reason why larger reductions in colitis were observed with video endoscopy, which visualizes the entire length of the colon versus pathology, where only four sections at 5 microns each are assessed during histopathology.

## Claims

1. A polyglucosamine-arginine (PAAG) of the Formula (I): 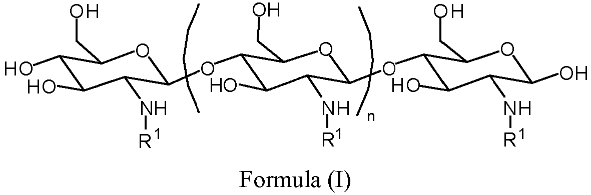 for use in a method of treatment of dysbiosis wherein:
n is an integer between 20 and 6000; and
each R¹ is independently selected for each occurrence from hydrogen, acetyl, 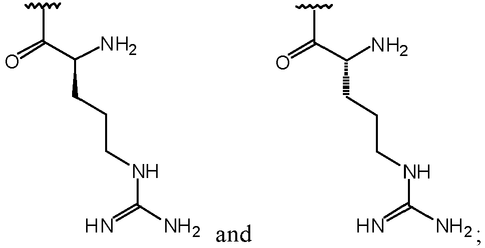
wherein at least 25% of R¹ substituents are H, and at least 2% of R¹ substituents are

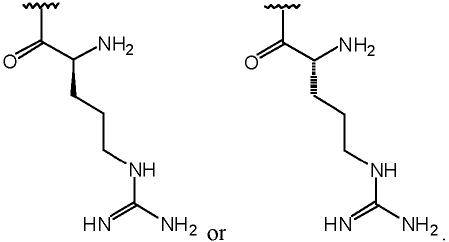

2. The PAAG for use in a method according to claim 1, wherein at least 1% of R¹ substituents are acetyl.

3. The PAAG for use in a method according to claim 1, wherein the method:
a. results in reduction or elimination of at least one pathogen or pathobiont present in the gastrointestinal (GI) tract of the subject;
b. results in augmentation or growth of at least one type of bacteria (e.g., at least one type of bacteria not detectably present in a composition comprising the PAAG or in the GI tract prior to administration of the PAAG in the subject);
c. provides microbiome homeostasis;
d. modulates the microbiota diversity present in the GI tract;
e. maintains or balances the diversity of the commensal microflora; or
f. reduces dissemination of bacteria (e.g., reduces by 20% inflammation scores from histology) to distal organs (e.g., liver, spleen, or lymph nodes).

4. The PAAG for use in a method according to claim 1, wherein the PAAG is administered:
a. concomitant to consumption of a food or beverage product;
b. prior to consumption of a food or beverage product; or
c. following consumption of a food or beverage product.

5. The PAAG for use in a method according to claim 1, wherein the dysbiosis is a result of:
a. an allergic effect;
b. an autoimmune and inflammatory disorder; or
c. celiac disease.

6. The PAAG for use in a method according to claim 1, wherein the method controls growth or colonization of the GI by one or more pathogenic bacterium.

7. The PAAG for use in a method according to claim 1, wherein the subject has been treated previously with an antibacterial or antibiotic agent.

8. The PAAG for use in a method according to claim 1, further comprising administration of an additional agent, preferably wherein the agent is synergistic with the PAAG.

9. The PAAG for use in a method according to claim 1, wherein the PAAG is delivered orally (e.g., as a dry product (e.g., capsule or tablet) or aqueous solution), or wherein the PAAG is delivered rectally (e.g., as an enema or suppository).

10. The PAAG for use in a method according to claim 1, wherein 10 to 5000 mg (e.g., 50 to 250 mg or 250 to 1000 mg) of the PAAG is administered to the subject, optionally daily.

11. The PAAG for use in a method according to claim 1, wherein the PAAG is administered:
a. once daily (e.g., for 1, 2, 3, or 4 weeks); or
b. 1, 2, 3, or 4 times at 4 to 50 mg/kg/dose/day.

12. The PAAG for use in a method according to claim 1, wherein the PAAG is administered as a composition additionally comprising:
a. a carrier, preferably wherein the carrier is osmotically balanced (e.g., with a neutral osmol); or
b. 1, 2, or 3% w/w or w/v carrier (e.g., glycerol); or
c. from 1 to 2% w/w or w/v glycerol.

13. The PAAG for use in a method according to claim 1, wherein the molecular weight average of the PAAG is
a. 20 to 200 kD, 20 to 150 kD, 30 to 120 kD, or 50 to 100 kD;
b. 25 to 80 kD;
c. 25 to 125 kD;
d. 25 to 70 kD;
e. 100 to 300 kD;
f. 20 to 350 kDa;
g. 50 to 120 kDa; or
h. 25 to 80 kDa.

14. The PAAG for use in a method according to claim 1, wherein the PAAG is arginine-functionalized:
a. at between 25 to 35%;
b. at between 15% and 40%;
c. at between 20% and 30%;
d. at between 25% and 35%;
e. at least 18%; or
f. at about 18% to about 40%.

15. The PAAG for use in a method according to claim 1, wherein the polydispersity index of the PAAG is from 1.0 to 2.5.

## Patentansprüche

1. Poly(acetyl, arginyl)glucosamin (PAAG) der Formel (I): 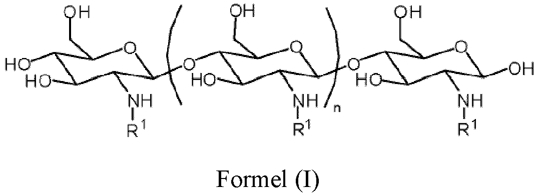 zur Verwendung in einem Behandlungsverfahren für Dysbiose, wobei:
n eine ganze Zahl zwischen 20 und 6 000 ist; und
jedes R¹ unabhängig ausgewählt ist für jedes Auftreten von Wasserstoff, Acetyl, 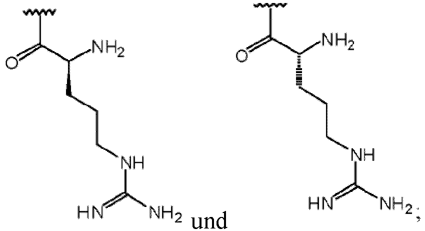
wobei mindestens 25 % von R¹-Substituenten H sind, und mindestens 2 % von R¹-Substituenten 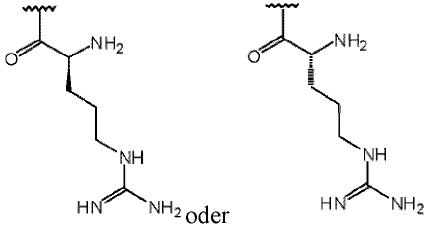 sind.

2. PAAG zur Verwendung in einem Verfahren nach Anspruch 1, wobei mindestens 1 % von R¹-Substituenten Acetyl sind.

3. PAAG zur Verwendung in einem Verfahren nach Anspruch 1, wobei das Verfahren:
a. zu einer Verringerung oder einer Beseitigung mindestens eines Pathogens oder Pathobionts führt, das im Gastrointestinal(GI)-Trakt des Probanden vorhanden ist;
b. zu einer Vermehrung oder einem Wachstum mindestens einer Bakterienart führt (z. B., mindestens einer Bakterienart, die in einer Zusammensetzung, die das PAAG umfasst, oder im GI-Trakt vor der Verabreichung des PAAG an den Probanden nicht nachweisbar vorhanden ist);
c. eine Mikrobiom-Homöostase bereitstellt;
d. die im GI-Trakt vorhandene mikrobielle Vielfalt moduliert;
e. die Vielfalt der kommensalen Mikroflora aufrechterhält oder ausgleicht; oder
f. eine Verbreitung von Bakterien in distale Organe (z. B. Leber, Milz oder Lymphknoten) verringert (z. B. verringert Entzündungswerte in der Histologie um 20 %).

4. PAAG zur Verwendung in einem Verfahren nach Anspruch 1, wobei das PAAG folgendermaßen verabreicht wird:
a. gleichzeitig mit dem Verzehr eines Nahrungsmittel- oder Getränkeprodukts;
b. vor dem Verzehr eines Nahrungsmittel- oder Getränkeprodukts; oder
c. nach dem Verzehr eines Nahrungsmittel- oder Getränkeprodukts.

5. PAAG zur Verwendung in einem Verfahren nach Anspruch 1, wobei die Dysbiose ein Ergebnis ist von:
a. einer allergischen Wirkung;
b. einer Autoimmun- und Entzündungserkrankung; oder
c. Zöliakie.

6. PAAG zur Verwendung in einem Verfahren nach Anspruch 1, wobei das Verfahren das Wachstum oder die Kolonisierung des GI durch ein oder mehrere pathogene Bakterien steuert.

7. PAAG zur Verwendung in einem Verfahren nach Anspruch 1, wobei der Proband zuvor mit einem antibakteriellen oder antibiotischen Mittel behandelt worden ist.

8. PAAG zur Verwendung in einem Verfahren nach Anspruch 1, ferner umfassend die Verabreichung eines zusätzlichen Mittels, vorzugsweise wobei das Mittel synergistisch mit dem PAAG ist.

9. PAAG zur Verwendung in einem Verfahren nach Anspruch 1, wobei das PAAG oral (z. B. als ein Trockenprodukt (z. B. Kapsel oder Tablette) oder eine wässrige Lösung) zugeführt wird, oder wobei das PAAG rektal (z.B. als Einlauf oder Zäpfchen) zugeführt wird.

10. PAAG zur Verwendung in einem Verfahren nach Anspruch 1, wobei 10 bis 5 000 mg (z. B. 50 bis 250 mg oder 250 bis 1 000 mg) des PAAG dem Probanden verabreicht werden, optional täglich.

11. PAAG zur Verwendung in einem Verfahren nach Anspruch 1, wobei das PAAG folgendermaßen verabreicht wird:
a. einmal täglich (z. B. über 1, 2, 3 oder 4 Wochen); oder
b. 1, 2, 3 oder 4 Mal mit 4 bis 50 mg/kg/Dosis/Tag.

12. PAAG zur Verwendung in einem Verfahren nach Anspruch 1, wobei das PAAG als eine Zusammensetzung verabreicht wird, die zusätzlich Folgendes umfasst:
a. einen Träger, vorzugsweise wobei der Träger osmotisch ausgeglichen ist (z. B. mit einem neutralen Osmosemittel); oder
b. 1, 2 oder 3 Massen-% oder Gew.-% Träger (z. B. Glycerin); oder
c. 1 bis 2 Massen-% oder Gew.-% Glycerin.

13. PAAG zur Verwendung in einem Verfahren nach Anspruch 1, wobei das Molekulargewichtsmittel des PAAG Folgendes ist:
a. 20 bis 200 kD, 20 bis 150 kD, 30 bis 120 kD, oder 50 bis 100 kD;
b. 25 bis 80 kD;
c. 25 bis 125 kD
d. 25 bis 70 kD;
e. 100 bis 300 kD;
f. 20 bis 350 kDa;
g. 50 bis 120 kDa; oder
h. 25 bis 80 kDa.

14. PAAG zur Verwendung in einem Verfahren nach Anspruch 1, wobei das PAAG Arginin-funktionalisiert ist:
a. zwischen 25 und 35 %;
b. zwischen 15 % und 40 %;
c. zwischen 20 % und 30 %;
d. zwischen 25 % und 35 %;
e. zu mindestens 18 %; oder
f. zwischen etwa 18 % und etwa 40 %.

15. PAAG zur Verwendung in einem Verfahren nach Anspruch 1, wobei der Polydispersitätsindex des PAAG zwischen 1,0 und 2,5 liegt.

## Revendications

1. Poly(acétyl, arginyl)glucosamine (PAAG) de formule (I) : 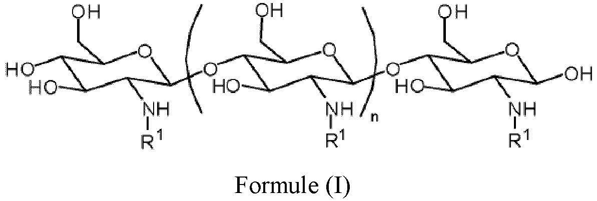 pour utilisation dans un procédé de traitement de la dysbiose, dans laquelle :
n est un nombre entier compris entre 20 et 6000 ; et
chaque R¹ est indépendamment choisi pour chaque occurrence parmi l'hydrogène, l'acétyle, 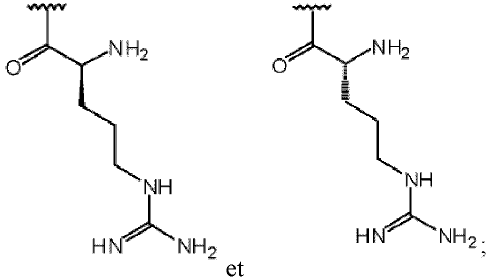
dans laquelle au moins 25 % des substituants R¹ sont H et au moins 2 % des substituants R¹ sont

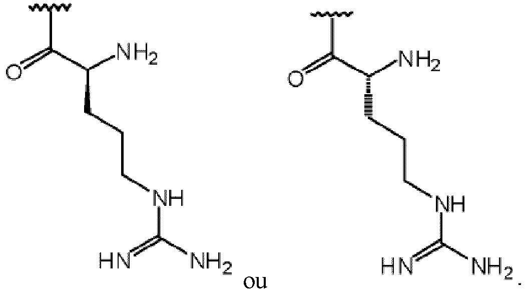

2. PAAG pour utilisation dans un procédé selon la revendication 1, dans laquelle au moins 1 % des substituants R¹ sont de l'acétyle.

3. PAAG pour utilisation dans un procédé selon la revendication 1, dans laquelle le procédé :
a. entraîne la réduction ou l'élimination d'au moins un agent pathogène ou pathobionte présent dans le tractus gastro-intestinal (GI) du sujet ;
b. entraîne l'augmentation ou la croissance d'au moins un type de bactérie (par exemple, au moins un type de bactérie, dont la présence n'est pas détectable dans une composition comprenant la PAAG ou dans le tractus GI avant l'administration de la PAAG au sujet) ;
c. assure l'homéostasie du microbiome ;
d. module la diversité du microbiote présent dans le tractus GI ;
e. maintient ou équilibre la diversité de la microflore commensale ; ou
f. réduit la dissémination de bactéries (par exemple, réduit de 20 % les scores d'inflammation de l'histologie) vers les organes distaux (par exemple, le foie, la rate ou les ganglions lymphatiques).

4. PAAG pour utilisation dans un procédé selon la revendication 1, dans lequel la PAAG est administrée :
a. de manière concomitante à la consommation d'un produit alimentaire ou de boisson ;
b. avant la consommation d'un produit alimentaire ou de boisson ; ou
c. après la consommation d'un produit alimentaire ou de boisson.

5. PAAG pour utilisation dans un procédé selon la revendication 1, dans lequel la dysbiose est un résultat :
a. d'un effet allergique ;
b. d'un trouble auto-immun et inflammatoire ; ou
c. de la maladie cœliaque.

6. PAAG pour utilisation dans un procédé selon la revendication 1, dans lequel le procédé contrôle la croissance ou la colonisation du GI par une ou plusieurs bactéries pathogènes.

7. PAAG pour utilisation dans un procédé selon la revendication 1, dans lequel le sujet a été traité précédemment avec un agent antibactérien ou antibiotique.

8. PAAG pour utilisation dans un procédé selon la revendication 1, comprenant en outre l'administration d'un agent supplémentaire, de préférence dans lequel l'agent est synergique avec la PAAG.

9. PAAG pour utilisation dans un procédé selon la revendication 1, dans lequel la PAAG est administrée par voie orale (par exemple, sous forme de produit sec (par exemple, capsule ou comprimé) ou de solution aqueuse), ou dans lequel la PAAG est administrée par voie rectale (par exemple, sous forme de lavement ou de suppositoire).

10. PAAG pour utilisation dans un procédé selon la revendication 1, dans lequel 10 à 5000 mg (par exemple, 50 à 250 mg ou 250 à 1000 mg) de la PAAG sont administrés au sujet, facultativement quotidiennement.

11. PAAG pour utilisation dans un procédé selon la revendication 1, dans lequel la PAAG est administrée :
a. une fois par jour (par exemple pendant 1, 2, 3 ou 4 semaines) ; ou
b. 1, 2, 3 ou 4 fois à raison de 4 à 50 mg/kg/dose/jour.

12. PAAG pour utilisation dans un procédé selon la revendication 1, dans lequel la PAAG est administrée sous la forme d'une composition comprenant de manière additionnelle :
a. un support, de préférence dans lequel le support est équilibré de manière osmotique (par exemple, avec une osmole neutre) ; ou
b. 1, 2 ou 3 % p/p ou p/v du support (par exemple, glycérol) ; ou
c. de 1 à 2 % p/p ou p/v de glycérol.

13. PAAG pour utilisation dans un procédé selon la revendication 1, dans lequel la masse moléculaire moyenne de la PAAG est de :
a. 20 à 200 kD, 20 à 150 kD, 30 à 120 kD ou 50 à 100 kD ;
b. 25 à 80 kD ;
c. 25 à 125 kD ;
d. 25 à 70 kD ;
e. 100 à 300 kD ;
f. 20 à 350 kDa ;
g. 50 à 120 kDa ; ou
h. 25 à 80 kDa.

14. PAAG pour utilisation dans un procédé selon la revendication 1, dans lequel la PAAG est fonctionnalisée à l'arginine :
a. à raison d'entre 25 et 35 % ;
b. à raison d'entre 15 % et 40 % ;
c. à raison d'entre 20 % et 30 % ;
d. à raison d'entre 25 % et 35 % ;
e. à raison d'au moins 18 % ; ou
f. à raison d'environ 18 % à environ 40 %.

15. PAAG pour utilisation dans un procédé selon la revendication 1, dans lequel l'indice de polydispersité de la PAAG vaut de 1,0 à 2,5.
